(19) Europäisches Patentamt
European Patent Office
Office européen des brevets

(11) EP 1 386 612 A1

(12) EUROPEAN PATENT APPLICATION
published in accordance with Art. 158(3) EPC

(43) Date of publication:
**04.02.2004 Bulletin 2004/06**

(21) Application number: **02718558.6**

(22) Date of filing: **12.04.2002**

(51) Int Cl.7: **A61K 31/661**, A61P 25/00, A61P 25/28, A61P 43/00

(86) International application number:
**PCT/JP2002/003659**

(87) International publication number:
**WO 2002/083149 (24.10.2002 Gazette 2002/43)**

(84) Designated Contracting States:
**AT BE CH CY DE DK ES FI FR GB GR IE IT LI LU MC NL PT SE TR**

(30) Priority: **13.04.2001 JP 2001115999**

(71) Applicant: **Gencom Corporation**
**Machida-shi, Tokyo 194-8511 (JP)**

(72) Inventors:
- **MUROFUSHI, Kimiko**
  **Saitama-shi, Saitama 336-0026 (JP)**
- **ASOU, Hiroaki**
  **Nishitokyo-shi, Tokyo 202-0013 (JP)**
- **KOBAYASHI, Tetsuyuki**
  **Itabashi-ku, Tokyo 174-0052 (JP)**

(74) Representative:
**Sternagel, Fleischer, Godemeyer & Partner**
**Patentanwälte**
**An den Gärten 7**
**51491 Overath (DE)**

# (54) DRUGS FO RPROMOTING THE PROLIFERATION, DIFFERENTIATION AND/OR SURVIVAL OF GLIAL CELLS CONTAINING CYCLIC PHOSPHATIDIC ACID

(57) An object of the present invention is to reveal an action of cPA on glial cells as a new physiological activity of cPA and to reveal the possibility of cPA as a medicament for treating a brain nerve disease including Alzheimer's disease, ischemic nerve disease, and Parkinsonism, and particularly cerebrovasucular dementia such as Binswanger's disease type dementia. According to the present invention, there is provided a medicament for treating cerebrovascular dementia, which comprises a cyclic phosphatidic acid derivative having a cyclic phosphoric acid structure at the sn-2 and 3 positions of glycerol.

EP 1 386 612 A1

**Description**

FIELD OF THE INVENTION

The present invention relates to a medicament which comprises a cyclic phosphatidic acid derivative, one of lysophospholipids. More particularly, the present invention relates to a medicament for promoting the proliferation, differentiation and/or survival of glial cells and a medicament for treating and/or preventing a nerve disease associated with the decrease of the glial cells, which comprise a cyclic phosphatidic acid derivative having an action on glial cells as an active ingredient, and a medicament for treating and/or preventing a nerve disease associated with decrease of the glial cells in number.

BACKGROUND ART

Glycerophospholipid, the main component of a biomembrane, has a glycerol skeleton which is coupled with two molecules of hydrophobic fatty acids and is bonded with a hydrophilic group such as cholin and ethanol amine via a phosphate group. A balance between the hydrophobic moiety and the hydrophilic moiety in the phospholipid is important for forming a stable lipid bilayer. On the other hand, lysophospholipid can not form a stable membrane structure and rather exhibits an action of a surface activity of destroying the same, because only one molecule of a fatty acid is bound thereto so that the lysophospholipid has relatively small hydrophobic portion as compared with hydrophilic groups.

However, many lysophospholipids which exhibit specific physiological activities at a low concentrations have been recently found, and one example thereof is lysophosphatidic acid (LPA). LPA is one of phospholipids having the simplest structure, and can be discriminated from phosphatidic acid (PA) in that fatty acid either at sn-1 or -2 positions of glycerol is deacylated (See Fig. 1).

LPA exists in a living body at a very small amount (0.5% or less of a total cellular phospholipid). LPA was understood to be an intermediate product or a decomposed intermediate in the biosynthesis of a phospholipid. But in the latter half of 1970s, a substance which exists in plasma (Schumacher, K.A., et al., Thromb. Haemostas., 42, 631-640 (1979)) or in a crude lecithin fraction from soy bean (Tokumura, A., et al., Lipids, 13, 468-472(1978)) and shows vasoconstrictive activity was identified to be LPA. Furthermore, it was also shown that a lipid growth factor in serum was LPA (van Corven, E., et al., Cell 59, 45-54(1989)), and LPA has attracted an attention as a physiologically active substance.

LPA has been demonstrated to have various physiological activities including cell proliferation promoting action (Fischer D.J.,et al., Mol Pharmacol, 54, 979-988 (1988)), promotion of infiltration of cancer cells (Imamura, F., et al.:Jpn.J.Cancer Res., 82, 493-496(1991); Imamura, F., et al.:Biochem. Biophys. Res. Commun., 193, 497-503(1993); and Imamura, F., et al.: Int. J. Cancer, 65, 627-632(1996)), inhibition of apoptosis (Umnaky, S.R, et al.: Cell Death Diff., 4, 608-616(1997)) and the like. Particularly, LPA is known to cause a recession of the neurodendrite of nerve cells (Tigyi, G, et al.:J. Biol. Chem., 267, 21360-21367(1992); Jalink, K., et al.: Cell Growth & Differ., 4, 247-255(1994); Jalink, K., et al.: J. Cell Biol., 126, 801-810(1994); and Tigyi, G, et al.: J. Nurochem., 66, 537-548(1996)). Also, LPA has been reported to induce opening release in PC12 cell, a nerve cell line (Shiono, S., et al.: Biochem. Biophys. Res Commun., 193, 663-667(1993)). Furthermore, in 1996, a gene of G protein-associated receptor (ventriluar zone gene-1;vzg-1/edg-2) which is specifically expressed in a nerve epithelial cell layer (ventriluar zone, vz) has been cloned by Chun et al., and from the finding that lipid in serum is required for the morphological change of the cells which overexpresses said gene, it was revealed that its specific ligand was LPA (Hecht, J. H., et al. :J. Cell Biol. 135, 1071-1083 (1996)). These observations suggest the importance of LPA signaling in a nerve system, and thus LPA is considered to play an important role in the development and differentiation of nerve.

The present inventors have made a cellular biochemical analysis using *Physarum Polycephalum*, a myxomycete, as an experimental material. The myxomycete has been demonstrated to take a morphological change depending on variation of external environment and take a proliferation/differentiation with a remarkable change in the composition and metabolism of a biomembrane lipid. A novel lipid component which was isolated and identified from a haploid myxoamoeba in 1992 was analyzed structurally, and was confirm to be a substance which contains hexadecanoic acid having a cyclopropnane ring at the sn-1 position of a glycerol skeleton, and is esterified with phosphoric acid to form a ring at the sn-2 and 3 positions (Murakami-Murofushi, K., et al.: J. Biol. Chem.,267, 21512-21517(1992)). This substance is named PHYLPA, since it is a LPA analog derived from the Physarum (See Fig.2).

PHYLPA is obtained from a lipid fraction which inhibits the activity of DNA polymerase a in a eukaryotic cell and suppresses the growth of an animal cultured cell, and PHYLPA is confirmed to show these physiological activities. PHYLPA has a characteristic fatty acid, but the structural analogues wherein this fatty acid moiety is replaced with other common fatty acid moieties were organically synthesized, and their physiological activities were studied to reveal that they had the similar activities to PHYLPA (Murakami-Murofushi, K., et al.: Biochem.Biophys.Acta, 1258, 57-60(1995)). Therefore, it is assumed that the cyclic phosphoric acid structure at the sn-2 and -3 positions of the glycerol is essential for these physiological activities. The lipid having this structure is generally called a cyclic phosphatidic acid (cPA) (see Fig. 2).

It was confirmed that cPA was not a lipid peculiar to myxomytes, but exists widely in living world. For example, the cPA having a palmitic acid (C16:0) residue in the fatty acid portion was isolated and identified from human serum albumin-bonded lipid, suggesting the existence of a small amount of cPA bonded with myristic acid (C14:0) and stearic acid (C18:0) residues. The concentration of cPA in serum is expected to be about $10^{-7}$ M, which equals to about one tenth of the concentration of LPA in serum (Kobayashi.T., et al.; Life Science, 65, 2185-2191(1999)). Thereafter, it was confirmed that cPA is present in human serum and rabbit lacrimal gland liquid, as in the case of LPA (Liliom, K., et al.: Am. J. Physiol., 274, C1065-1074(1998)).

cPA has been reported to exhibit various physiological activities which are contrary or similar to those of LPA. For examples, cPA has been reported to inhibit a cell growth (Murakami-Murofushi, K., et al.: Cell Struct. Funct., 18, 363-370(1993)), to inhibit invasion of cancer cells (Mukai, M., et al.:Int.J.Cancer, 81, 918-922, 1999), and to form a stress fiber within cells (Fischer, D.J.,et al.: Mol.Pharmacol., 54, 979-988(1998)).

The senescence and dysfunction of glial cells with aging have a close relation with senile dementia. Further, recent observations have revealed that, in geriatric brains, glial cells decrease significantly rather than neurons and particularly myelins are subjected to damage and decrease. It is demonstrated that in Binswanger's disease type dementia, a cerebrovascular dementia often found in Japan, a blood stream in brain is lowered and a leukomyelin sheath is first damaged. However, there has been no report on the action of cPA on glial cells.

SUMMARY OF THE INVENTION

A problem to be solved by the present invention is to reveal the action on glial cells as one of novel physiological activities of cPA and to provide a novel medicament which is useful for treating and/or preventing a nerve disease associated with decrease of glial cells by promoting proliferation, differentiation and/or survival of the the glial cells.

The present inventors have made diligent studies to solve the above problem. As a result, it has been revealed that cPA promotes the proliferation and survival of an oligodendrocyte which plays a main role in forming a myelin, and that cPA also contributes to promote the survival of the oligodendrocyte by promoting the survival of an astrocyte which supplies the oligodendrocyte with nutrient. Based on these observations, the present inventors have found that cPA can be a medicament for treating and/or preventing a nerve disease (including a cerebrovascular dementia such as Binswanger's disease type dementia) associated with decrease of glial cells, and thus the present invention has been completed.

Namely, according to the present invention, there is provided a medicament for promoting the proliferation, differentiation and/or survival of glial cells, which comprises a cyclic phosphatidic acid derivative represented by the formula (I) below as an active ingredient:

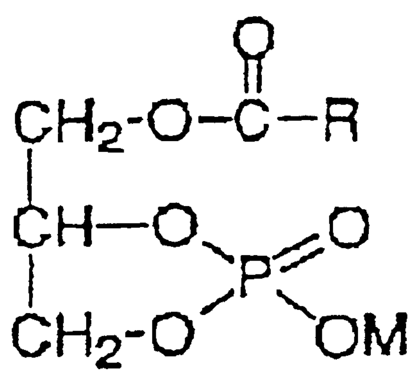

wherein R is a $C_{1-30}$ linear or branched alkyl group, a $C_{2-30}$ linear or branched alkenyl group, or a $C_{2-30}$ linear or branched alkynyl group, wherein these groups may contain a cycloalkane ring or an aromatic ring; and M is a hydrogen atom or a counter cation.

The glial cell is preferably a cell selected from an oligodendrocyte, a Schwann cell, an astrocyte or a microglia, and particularly preferably a cell selected from an oligodendrocyt or an astrocyte.

According to another aspect of the present invention, there is provided a medicament for treating and/or

preventing a nerve disease associated with decrease of the glial cells which comprises a cyclic phosphatidic acid derivative represented by the formula (I) above as an active ingredient.

The nerve diseases associated with decrease of the glial cells include cerebrovascular dementia, and specific examples thereof include Binswanger's disease type dementia.

The cyclic phosphatidic acid derivative represented by the formula (I) is particularly preferably 1-oleoyl cyclic phosphatidic acid.

According to further another aspect of the present invention, there are provided a method for promoting the proliferation, differentiation and/or survival of glial cells which comprises administrating a therapeutically effective amount of the cyclic phosphatidic acid derivative represented by the formula (I) above to a mammal including human; and a method for treating and/or preventing nerve diseases associated with decrease of the glial cells which comprises administrating a therapeutically effective amount of the cyclic phosphatidic acid derivative represented by the formula (I) above to a mammal including human.

According to further another aspect of the present invention, there are provided an use of the cyclic phosphatidic acid derivative represented by the formula (I) above in the production of a medicament for the proliferation, differentiation and/or survival of glial cells; and an use of the cyclic phosphatidic acid derivative represented by the formula (I) above in the production of a medicament for treating and/or preventing nerve diseases associated with decrease of the glial cells.

BRIEF DESCRIPTION OF DRAWINGS

Fig.1 shows the structure of phosphatidic acid (PA) and lysophosphatidic acid (LPA).

Fig.2 shows the structure of lysophospholipids. "A" shows 1-acyl LPA, "B" shows PHYLPA, and "C" shows 1-acyl cPA.

Fig.3 is a diagram which explains the type and function of the brain nerve cells.

Fig.4 is a diagram which explains the differentiation of a glial cell.

Fig.5 is a diagram which explains a variety of specific proteins which are expressed at various stages of an oligodendrocyte.

Fig.6 shows a diagram which briefly shows the primary culturing of the oligodendrocyte, and the cells on the culturing process.

Fig.7 shows cell images at the 6th day from the start of culturing in a control sample, a cPA addition sample and a LPA addition sample.

Fig.8 shows cell images at the 6th day from the first passage in the control sample, the cPA addition sample and the LPA addition sample.

Fig.9 shows cell images at the 6th day from the second passage in the control sample, the cPA addition sample and the LPA addition sample.

Fig.10 shows stained cell images at the 6th day from the second passage in the control sample, the cPA addition sample and the LPA addition sample.

Fig.11 shows cell images at the 6th day from the third passage (3P-OL) in the control sample, the cPA addition sample and the LPA addition sample.

Fig.12 shows stained cell images at the 6th day from the third passage (3P-OL) in the control sample, the cPA addition sample and the LPA addition sample.

Fig.13 shows cell images on further 10 days after the arrival at 3P-OL.

BEST MODE FOR CARRYING OUT THE INVENTION

Hereafter, embodiments of the present invention will be described in detail.

A medicament of the invention can be used for promoting the proliferation, differentiation and/or survival of glial cells as well as for treating and/or preventing nerve diseases associated with decrease of the glial cells, and the medicament comprises a cyclic phosphatidic acid derivative represented by the formula (I) below as an active ingredient:

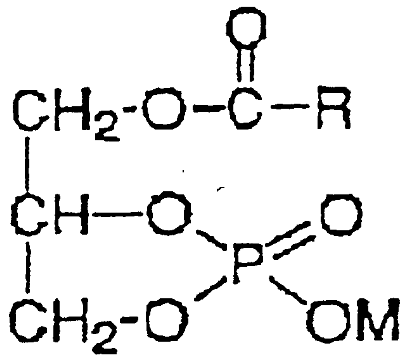

wherein R is a $C_{1-30}$ linear or branched alkyl group, a $C_{2-30}$ linear or branched alkenyl group, or a $C_{2-30}$ linear or branched alkynyl group, wherein these groups may contain a cycloalkane ring or an aromatic ring; and M is a hydrogen atom or a counter cation.

Examples of the $C_{1-30}$ linear or branched alkyl groups represented by the substituent R in the formula (I) include a methyl group, an ethyl group, a propyl group, a butyl group, a pentyl group, a hexyl group, a heptyl group, an octyl group, a nonyl group, a decyl group, a pentadecyl group, and an octadecyl group.

Examples of the $C_{2-30}$ linear of branched alkenyl group represented by the substituent R include an allyl group, a butenyl group, an octenyl group, a decenyl group, a dodecadienyl group, and a hexadecatrienyl group. More specifically, the examples include 8-decenyl group, 8-undecenyl group, 8-dodecenyl group, 8-tridecenyl group, 8-tetradecenyl group, 8-pentadecenyl group, 8-hexadecenyl group, 8-heptadecenyl group, 8-octadecenyl group, 8-icocenyl group, 8-dococenyl group, heptadeca-8,11-dienyl group, heptadeca-8,11,14-trienyl group, nonadeca-4,7,10,13-tetraenyl group, nonadeca-4,7,10,13,16-pentaenyl group, and henicosa-3,6,9,12,15,18-hexaenyl group.

The examples of the $C_{2-30}$ linear or branched alkynyl group represented by the substituent R include 8-decynyl group, 8-undecynyl group, 8-dodecynyl group, 8-tridecynyl group, 8-tetradecynyl group, 8-pentadecynyl group, 8-hexadecynyl group, 8-heptadecynyl group, 8-octadecynyl group, 8-icocynyl group, 8-dococynyl group, and heptadeca-8,11-diynyl group.

The examples of the cycloalkane ring which may be contained in the above described alkyl group, alkenyl group or alkynyl group include, for example, a cyclopropane ring, a cyclobutane ring, a cyclopentane ring, a cyclohexane ring, and a cyclooctane ring. The cycloalkane ring may contain one or more hetero atoms, and examples thereof include an oxylane ring, an oxetane ring, a tetrahydrofuran ring, and an N-methylprolidine ring.

The examples of an aromatic ring which may be contained in the above described alkyl group, alkenyl group or alkynyl group include, for example, a benzene ring, a naphthalene ring, a pyridine ring, a furan ring, and a thiophene ring.

Accordingly, in the case where the substituent R is an alkyl group substituted with a cycloalkane ring, the examples include a cyclopropylmethyl group, a cyclohexylethyl group, and an 8,9-methanopentadecyl group.

In the case where the substituent R is an alkyl group substituted with an aromatic ring, the examples include a benzyl group, a phenetyl group, and a p-pentylphenyloctyl group.

M in the cyclic phosphatidic acid (cPA) derivative represented by the formula (I) is a hydrogen atom or a counter cation. In the case where M is a counter cation, examples thereof include an alkali metal atom, an alkali earth metal atom, and a substituted or unsubstituted ammonium group. The alkali metal atom includes, for example, lithium, sodium and potassium. The alkali earth metal atom includes, for example, magnesium and calcium. The substituted ammonium group includes, for example, a butylammonium group, a triethylammonium group and a tetramethylammonium group.

As a specific example of the cPA represented by the formula (I) which is used in the present invention, an oleoyl cPA is particularly preferable.

The cPA derivative represented by the formula (I) can be chemically synthesized according to the methods disclosed in, for examples, Japanese Patent Laid-open Publications JP-A-5-230088, JP-A-7-149772, JP-A-7-258278, and JP-A-9-25235.

Alternatively, the cPA derivative represented by the formula (I) can also be synthesized by reacting the lysophospholipid with phospholipase D according to the method described in Japanese Patent Application No. 367032/1999. The lysophospholipid used here is not limited, so far as it can be reacted with phospholipase D. Many types of lysophospholipids are known, including those which are different in fatty acid and molecular species which have an ether or vinylether bond. They are available in the market. As for the phospholipase D, those derived from a higher plant such as cabbage and peanut or from a microorganism such as *Streptomyces chromofuscus* and *Actino-*

*madula sp*. are available in the market. cPA can be highly selectively synthesized with the enzyme derived from *Actinomadula sp*. No.362 (Japanese Patent Laid-open Publication JP-A-11-367032). Any condition may be available without limitation for reacting the lysophospholipid with the phospholipase D, as far as it allows the enzyme to exhibit the activity. The reaction of the lysophospholipid with the phospholipase D is carried out, for example, in an acetate buffer (around pH 5-6) containing calcium chloride at room temperature to a warmed temperature (preferably about 37°C) for around 1-5 hours, although the condition of the reaction is not particularly limited so far as the condition allows the expression of the enzyme activity. The thus produced cPA derivative may be purified by extraction, column chromatography, thin layer chromatography (TLC) or the like according to a conventional method.

Next, the glial cell which is a target of the action of the medicament according to the invention will be described.

The senescence or dysfunction of glial cells which help work and maintenance of neurocyte due to aging, has a close relation with senile dementia. In a geriatric brain, glial cells decreases remarkably and, particularly, myelins are damaged and decreased in number significantly.

It is an oligodendrocyte that plays a main role in the formation of the myelin in a central nervous system. Clarification of the family tree of the oligodendrocyte in a normal brain and an aging brain and its dysfunction not only gives a key for axon regeneration but also leads to recovery of functions of damaged nerve cells.

In the Example described later, as a part of clarification of dysfunctions, it is tried to clarify the action of a functional phospholipids cPA on oligodenderocytes and astrocytes, and the mechanism of intracellular response; and it is intended to develop methods for treating and preventing brain diseases such as brain nerve disease and senile dementia while focusing on clarification of the proliferation and differentiation of the oligodenderocytes, and its myelin forming ability.

Firstly, the constitution, differentiation and function of brain nerve cells will be described with reference to the drawings. As shown in Fig.3, in the brain nerve, various types of cells are combined to express high dimensional functions. The glial cells differentiate from the neuroepithelial cells, exist far more than the neurocytes in number, and support the work and maintenance of the nerve cells. In a higher animal, the precursor cells of neurons or glia cells differentiate to neurons or glia depending on the surrounding conditions with the maintained multipotentiality in differentiation. Fig. 4 shows such differentiations.

As shown in Fig.4, some astrocytes grow through radial glia, and the others grow not through radial glia. The O-2A precursor cells can differentiate to astrocytes in the presence of serum. The astrocytes function to supply nerve cells such as oligodendrocytes and neurons with energy. For example, the astrocytes secrete a growth factor such as PDGF and bFGF which act on the O-2A precursor cells. The astrocyte cells express a glia fibrous acidic protein (GFAP) and the A2B5 protein as a surface antibody. Since GFAP is specific to the astrocytes, it can be used as an index in the cell staining of the astrocytes.

The oligodendrocytes differentiate from the O-2A precursor cells. The O-2A precursor cells have the A2B5 as the surface antibody, and grow in response to the growth factor such as PDGF and bFGF which are secreted from the astrocytes. The cells can differentiate to oligodendrocytes in the absence of serum.

As shown in Fig.5, the oligodendrocytes (OL) express various specific proteins depending on the differentiation stage. Furthermore, PDGF and bFGF also work specifically depending on the differentiation stage. In the matured oligodendrocytes, dendrites in a mesh form are extended toward axons, and are wound around the axons to form myelin sheaths.

The medicament for promoting the proliferation, differentiation and/or survival of glial cells according to the invention can promote the survival of oligodendrocytes which play a main role in the formation of myelin sheaths to protect nerve cells and contribute the promotion of survival thereof. Thus, the medicament can treat a brain nerve disease in cerebrovascular dementia such as senile dementia, and can contribute to recover functions of damaged nerve cells through the actions as mentioned above.

The medicament for promoting the proliferation, differentiation and/or survival of glial cells according to the present invention can promote the survival of the myelination responsible cells by the actions as described above, to promote the supply of nutrition to brain and protect the brain from senescence.

The medicament of the present invention is preferably provided in the form of a pharmaceutical composition which comprises one or more pharmaceutically acceptable additives and the cPA derivative represented by the formula (I) as an active ingredient.

The medicament of the present invention can be administered in various forms, and preferably has a form capable of passing through a blood-brain barrier, since its main active site is brain. Such suitable dosage forms may be peroral or parenteral (for examples, intravenous, intramuscular, subcutaneous or intracutaneous injection, rectal dosage, and permucosal dosage). Examples of the pharmaceutical composition suitable for peroral dosage include a tablet, a granule, a capsule, a powder, a solution, a suspension, and a syrup. Examples of the pharmaceutical composition suitable for parenteral dosage include an injection, an infusion, a suppository, and a percutaneous absorption agent. The dosage form of the medicament of the present invention is not limited to these. Furthermore, the medicament of the present invention can also be made into sustained release formulations by publicly known methods.

The type of the pharmaceutical additives used for producing the medicament of the present invention is not particularly limited, and can be suitably selected by a person skilled in the art. For examples, one can use an excipient, a disintegration agent or a disintegration auxiliary agent, a binder, a lubricant, a coating agent, a base, a solvent or a solubilizer, a dispersant, a suspension agent, an emulsifier, a buffer, an antioxidant, an antiseptic, an isotonic agent, a pH adjusting agent, a solving agent, and a stabilizer. Individual ingredients which are used for the above purposes are well known to a person skilled in the art.
Examples of the pharmaceutical additives usable for preparing a peroral preparation include an excipient such as glucose, lactose, D-mannitol, starch and crystalline cellulose; a disintegration agent or a disintegration auxiliary agent such as carboxymethyl cellulose, starch and carboxymethyl cellulose calcium; a binder such as hydroxypropyl cellulose, hydroxypropyl methylcellulose, polyvinyl pyrrolidone, and gelatin; a lubricant such as magnesium stearate and talc; a coating agent such as hydroxypropyl methylcellulose, white sugar, polyethylene glycol and titanium oxide; a base such as Vaseline, liquid paraffin, polyethylene glycol, gelatin, kaolin, glycerin, purified water, and hard fat.
Examples of the pharmaceutical additives which can be used for preparing an injection or an infusion preparation include a solvent or a solubilizer which can be used for an aqueous injection or a use-time dissolution type injection such as injection distilled water, physiological saline, and propylene glycol; an isotonic agent such as glucose, sodium chloride, D-mannitol, and glycerin; and a pH adjusting agent such as an inorganic acid, an organic acid, an inorganic base and an organic base.
The medicament of the present invention can be administered to a mammal including human.
The dose of the medicament of the present invention should be increased or decreased according to the conditions such as age, sex, body weight, symptom of a patient, and dosage route. The dose of the active ingredient per day for an adult is generally 1μg/kg to 1,000mg/kg, and preferably 10μg/kg to 100mg/kg. The medicament of the dose as mentioned above may be administered once a day, or may be dividedly administered a few times (for example, about 2-4 times) a day.
The medicament of the present invention may be used in combination with another medicament which is effective for treating or preventing a nerve disease, a nutrient for supplying brain nerve with energy, or the like.
As is obvious from Example 1 as described later, cPA itself is a substance which exists in brain of mammals, and is considered to be safe to a living body.
All the disclosures in Japanese Patent Application No.115999/2001, which the present application claims priority based on, shall be disclosed herein by reference.
The present invention will be described in detail with reference to the following Examples, but the present invention is not limited by the Examples.

EXAMPLES

Example 1: Biosynthesis of cPA using phospholipase D (PLD)

In Example 1, it was revealed that cPA can be generated from lysophosphatidyl cholin (LPC) using an actinomyces-derived PLD, and that an enzyme for generating cPA exists in the brain of mammalian.

(A) Material and method

(A-1) Experiment material

PLD derived from an actinomyces, *Streptomyces chromofuscus* (*S.chromofuscus*), and PLD derived from cabbage were purchased from Sigma. PLD derived from *Actinomadura sp.* No. 362 (*A. sp.* No.362) was purchased from the Meito Sangyo. 1-oleoyl LPC and lysophosphatidylserine (LPS) were purchased from Avanti Polar lipid, INC. Lysophosphatidylethanolamine (LPE) was purchased from Doosan Serdary Res. Lad. 1-alkyl lysophosphatidylcholine (1-alkyl LPC) and 1-alkenyl phosphatidylcholine plasmalogen (1-alkenyl LPC) were purchased from Sigma. Oleoyl cPA which was synthesized according to the method described in Kobayashi, S., et al.: Tetrahedron Lett., 34, 4047-4050 (1993) was also used.

(A-2) Synthesis of 1-NBD-LPC

A fluorescence labeled LPC was prepared as a substrate for determining the cPA generating activity. Namely, by using 1-hexadecanoyl-2-(12-(7-nitrobenz-2-oxa-1,3 -diazol-4-yl))-sn-glycero-3-phosphocholin (2-NBD-HPC; made by Avanti Polar lipids, INC.) as a starting material, a fatty acid only at 1-position was deacylated with a lipase (derived from *Rhizopus delemer*, made by Seikagaku Kogyo), then a fluorescence labeled acyl group at 2-position was displaced to 1-position in Tris-HCl buffer (pH=9), and the product was purified by high performance liquid chromatography (HPLC)

to obtain 1-NBD-LPC.

(A-3) Assay of cPA generating activity

As the substrate, a mixture of 1-NBD-LPC and egg yolk derived LPC at the ratio of 1:99 was used (1% NBD-LPC). The enzyme assay was carried out in 100mM acetate buffer (pH=5.6) containing 10mM calcium chloride in the presence of 100μM of 1% NBD-LPC. For the enzyme source, 2.2μg/ml of PLD derived from an actinomyces, *S. chromofuscus,* or 1.4μg/ml of PLD derived from an actinomyces, *A. sp.* No.362, was used. The reaction was carried out at 30°C (in the case of *S.chromofuscus*) or at 37°C (in the case of *A. sp.* No. 362). At the end of the reaction, 0.3 times volume of 0.1M citric acid solution was added to the reaction solution to make it acidic, and then 5.4 times amount of a chloroform:methanol (2:1) mixed solution was added, followed by centrifuging (1,400 x g, 5 minutes) to extract a lipid in the lower layer. The same extraction with the chloroform:methanol (2:1) mixed solution was repeated once again, and the obtained lower layers were mixed and concentrated to dry under a nitrogen stream. The thus obtained lipid was dissolved again in a small amount of the chloroform:methanol (2:1) mixed solution, and was spotted on a Silica Gel 60F thin layer chromatography plate (TLC; made by E. Merck). Using a developing solvent: chloroform/ methanol/acetic acid/5% aqueous sodium bisulfite solution (100:40:12:5), the lipid was separated, and the intensity of each fluorescent spot was quantified by a fluoroimage analyzer, FLA-2000 (made by Fuji Photo Film).

(A-4) Structural analysis by ESI-MS/MS

By using an apparatus manufactured by connecting Quattro II (made by the Micromass), which was a Tandem quadrupole type mass spectrometer equipped with an electrospray type ion source, with HPLC, a sample was analyzed in an anionic mode. By using Hewlett Packerd model 1050 HPLC pump (made by Hewlett Packerd), the sample was eluted with an acetonitrile:methanol (1:1) mixed solution at a flow rate of 5μg/min. 3-5μl of the sample, which contained lipid dissolved to be a concentration of 10-50 pmol/μl in the acetonitrile/methanol (1:1) mixed solution containing 0.1% ammonium formate, was injected. Formic acid and ammonia work as a proton donor or acceptor when the sample was ionized, respectively. The interface between the HPLC and the MS was maintained at 80°C, and nitrogen gas for purging the solvent was set at a pressure of 40 psi and a flow rate of 0.4 1/min. In the MS analysis, a molecular ion was monitored at a cone voltage of -30eV. In the MS/MS analysis, a fatty acid was monitored at a cone voltage of -90eV, and and phosphoric acid was monitored at a cone voltage of -170eV. In the MS/MS analysis, a technique was combined where a high pressure is locally formed by introducing an inert gas, and daughter ions were generated by collision induced dissociation (CID) of molecule ions. Argon (pressure 3.0-4.5$e^{-4}$ Torr) was used as the collision gas, and the collision energy was set to be -50eV.

(A-5) Formation of stress fiber in NIH-3T3 by addition of various lysophospholipids

NIH-3T3, a mouse-derived fibroblast cell, was incubated in a Dulbecoo's Modified Eagle's Medium (DMEM) containing 10% fetal bovine serum (FBS; made by Moregate), and was used in the Experiment. 2.5x$10^4$ cells of the NIH-3T3 were inoculated in a petri dish (10cm in diameter) paved with cover glasses of 22mm in diameter, and the medium was replacing with a FBS-free medium after 24 hours, and the cells were incubated in a serum starvation state for 48 hours. 10μM of the lysophospholipid was added, and the cells were incubated at 37°C for 30 minutes for stimulation. Then, the cells were fixed in the Dalbecco's PBS containing 3.7% of paraformaldehyde and 0.1% of Triton X-100 at room temperature for 10 minutes. Thereafter, the cells were stained with 5 units/ml of rhodamine phalloidin (made by Funakoshi) at 37°C for 1 hour. The cover glasses were washed with PBS thrice, and observation was carried out by a cofocus laser microscope TCS NT Control laser Scanning Microscope (made by Leica).

(A-6) Assay of cPA generating activity in rat brain

Male 4-weeks old Sprague-Dawley rats were employed for subjects. The rats were anesthetized with ether and decapitated to separate their whole brains, which were preserved at -80°C. Either left or right half side (about 0.8g) of the rat whole brains thus preserved was added with 10 times volume of 0.32M sucrose solution, and was homogenized twice for 20 seconds by the Polytron Homogenizer (made by Polytron) at a power control of 7. Twice volume of Hepes buffer was added thereto to prepare a homogenate solution. The assay of cPA generating activity was carried out in the following solution composition. Namely, 1%NBD-LPC 40nmol or a mixture (120nCi) of $^{14}C$-LPC which was radiation-labeled at the carbon in the carboxyl group of the fatty acid at 1-position, and an unlabeled LPC at a ratio of 2:55 was used as a substrate, and it was reacted in 100μl of the homogenate solution in the presence of 450μM of sodium oleate at 37°C. As for process after the completion of the reaction, the conditions for the actinomyces PLD assay were applied.

(B) Results

(B-1) Generation of LPA/cPA by actinomyces PLD

It was studied whether the generation of cPA could be found or not by using two types of actinomyces-derived PLDs which were different in the intensity of phosphate displacement activity and using 1%1-NBD-LPC as a substrate. In the case where the *S.chromofuscus*-derived PLD was used, the reaction of 20 minutes yielded only 1-NBD-LPA as the main product. On the other hand, in the case where the A.sp.362-derived PLD which was considered to have a high phosphate displacement activity was used, a product different from LPA was mainly obtained, and the Rf value of this compound coincided with that of the organic-synthesized cPA standard (oleoyl cPA). In order to confirm the difference between these reactions, the enzyme level dependency and the reaction time dependency were studied respectively. In the case where the *S.chromofuscus*-derived PLD was used, only the generation of LPA was observed with decrease of LPC which is the substrate. In contrast, in the case of the A.sp.362-derived PLD was used, only increase of product corresponding to cPA was observed, and almost no generation of LPA was observed. These observations shows that different types of LPDs generates different products regardless of the same substrate. In the case where the cabbage-derived PLD, which have been well analyzed enzymologically, was used, both LPA and cPA were generated at a rate of about 6:4.

Here, the A.sp.362-derived PLD, which generated a cPA-corresponding compound, was further studied with respect to substrate specificity. By using each 100μM of 1-acyl LPC, 1-alkyl LPC, 1-alkenyl LPC, LPS, and LPE as a substrate, reaction was carried out according to the standard PLD assay condition. The product from respective substrates was separated by TLC, and the spots corresponding to LPA and cPA were collected, and the generation amount was determined by phosphor quantification. Almost no generation of LPA was observed for both substrates. In the case where LPC, 1-alkyl LPC or 1-alkenyl LPC was used as a substrate, cPA generated time-dependently, while cPA was not generated when LPS and LPE were used as the substrate. From these results, it was showed that the A.sp. 362-derived PLD generated cPA effectively from the lysophospholipid having a choline at the polar group moiety. Additionally, alkyl and alkenyl type LPC were also found to be able to be a substrate for cPA generation.

(B-2) Structural analysis of a reaction product by *A.sp*.362-derived PLD

Mass spectrometry was utilized to perform structural analysis of the main product (a compound having the same Rf value as that of cPA) obtained by reacting, a substrate, 1-oleyl LPC having oleic acid as the fatty acid at 1-position, with A.*sp*.362-derived PLD. Firstly, in order to set the condition for the mass spectrometry, organically synthesized 1-oleoyl cPA was analyzed as a standard by ESI-MS/MS in an anionic mode. As a result, a peak of m/z 417 corresponding to the molecular ion $[M-H]^-$ of the 1-oleoyl cPA was observed under the above described condition. When the PLD reaction product was analyzed under the same condition, a strong peak of m/z 417 was similarly observed. In order to obtain more structural information. tandem mass spectrometry (MS/MS) by in-source fragmentation was carried out on a group of peaks of the molecular ion. As a result of daughter scanning using a molecular ion m/z 417 of the standard cPA as the parent ion, several characteristic ion peaks were generated. It was understood that m/z 281, m/z 153, and m/z 79 were attributed to $C_{17}H_{33}COO^-$, $[M-C_{17}H_{33}CO]^-$, and $PO_3^-$ respectively. On the other hand, the m/z 417 peak obtained by the PLD reaction was similarly analyzed by MS/MS, and as a result, a group of the identical characteristic fragment peaks was observed. From the aforementioned result, it was confirmed that the compound generated by the *A.sp*.362-derived PLD reaction was cPA.

(B-3) Formation of stress fiber in NIH-3T3 cell by PLD reaction product

The product obtained by the A.sp.362-derived PLD reaction was studied with respect to its biochemical activity in order to confirm further that it was a compound which is identical to cPA. Namely, the activity of forming actin stress fiber in a fibroblast cell, one of the physiological acteivities of cPA, was studied. To NIH-3T3, i.e. a fibroblast cell line derived from mouse, in the subconfluent state in the serum starvation state, each 10μM of LPA, a chemically synthesized PHYLPA, or cPA, i.e. the PLD reaction product was added at 37°C for 30 minutes, and then actin stress fiber was stained with Rhodamine phalloidin, and observation was carried out. In the control cells without addition of the lipid, no formation of stress fiber was observed, while the formation of stress fibers was observed in the case of addition of either one of the three types of lipids.

(B-4) Generation of cPA in rat brain

Possibility of existence of cPA generating activity in rat brain was studied. As the result of studies under several different conditions, finally homogenate was prepared with an aqueous sucrose solution (0.32M), and the

activity was determined in the Hepes buffer (pH 7.2) in the presence of oleic acid (450μM). As a result, cPA corresponding spots were confirmed after incubation at 37°C for 60 minutes.

(C) Conclusion

From the above results, it was showed that cPA could be generated by a specific enzyme among phospholipid hydrolases generally called PLD. It was revealed that even using PLDs available in the market as purified products, if its enzyme source was different, a different product was obtained when LPC was used as a substrate.

The finding of Example 1 that the enzymatic activity for generating cPA from LPC was detected in the rat brain homogenate, shows that phosphatidyl group displacement reaction contributes positively to the production of cPA which is a physiologically active lipid. A mammalian brain has been demonstrated to contain cPA and also have a relatively high PLD activity. The substrate LPC is scarcely detected in brain under a normal physiological condition, but is considered to be generated through activation of a certain type of $PLA_2$.

It is useful for producing cPA-structural homologues that cPA can be prepared effectively by the use of the actinomyces *A.sp.*362-derived PLD. In the preparation method using such an enzyme, cPA can be prepared from 1-alkenyl LPC. 1-alkenyl LPA is detected in the injury of a rabbit cornea, has an activity for proliferating cells, and is involved in healing of a wound. In addition, the corresponding LPAs/cPAs can be prepared from LPCs which are different in fatty acid.

Example 2: Establishment of primary culture of oligodendrocyte

For maturing an immature oligodendrocyte, it is necesary to carry out co-culturing by direct contact with an astrocyte. Oligodendrocytes differentiate via co-culturing with an astrocyte, and can be matured into myelin formation-responsible cells. If co-cultured with neurons, they remains immature. Since the oligodendrocytes are cells which form a very uneven population, the large scale preparation of the precursor cells is a large problem in the culturing. In view of these, a method for culturing the oligodendrocyte was firstly established as follows.

(1) Preparation of culture medium and reagent

(i) $MEM^-$(no serum added)

4.7g of a powdery Eagle MEM medium was dissolved in pure water, followed by sterilization under a high pressure in 121°C autoclave for 15 minutes and cool down to room temperature. 5.0ml of 40g/ml glucose solution and 5.0ml of 2.92g/100ml L-glutamine solution (preserved at -20°C in a refrigerator) were added thereto, and then $NaHCO_3$ was added to make pH 7.4-7.6, and the resultant was stored at 4°C in a refrigerator.

(ii) $MEM^+$(with serum added)

Fetal bovine serum (FBS) was added to $MEM^-$ to be the concentration of 10%, and the resultant was stored at 4°C in a refrigerator.

(iii) $PBS^-$

4.8g of Dulbecco's PBS (-) powder was dissolved in pure water, followed by sterilization under a high pressure in a 121°C autoclave for 15 minutes and cool down to room temperature, and the resultant was stored at 4°C in a refrigerator.

(iv) B.S medium (serum-free culture medium) was stored at 4°C in a refrigerator.

(2) Preparation in previous day

0.02mg/ml poly-L-lysine (PLL) solution was added in dishes at about 3ml/dish (10cm), and the dishes were left to stand in a clean bench. When PLL coating was performed on experiment day, it was similarly added in the dishes and the dishes were set in an incubator for about 15-30 minutes. On the experiment day, the dishes were washed twice with pure water before inoculation of the cells.

(3) Experiment procedure

A mother rat of 18 days of pregnancy (El8Wistar Rat) was put into an anesthesia bottle containing an adequate amount of diethyl ether, and was anesthetized. The anesthetization was carried out for 3-5 minutes and stopped just before death. The mother rat was taken out of the anesthesia bottle, put on a suitable vat to lie on its back, and disinfected at the hypogastric area with ethanol. The rat was torn along a cut line about 3-4cm upward from the tail joint by a scissors, and opened sideward with the scissors to separate the inner membrane from the skin. The skin was torn to expose the inner membrane, which was disinfected again with ethanol and torn to take out the uterus. The Y-shaped section of the uterus was cut while lifting the uterus, and the uterus was taken out. The taken out uterus was dipped in a cooled $MEM^-$ in order to decrease the damage of the fetus brain.

All the following operations were carried out in a clean bench in an aseptic room. The fetus, which was wrapped in the double membranes of the uterus, was taken out of them and was dipped in a cooled $MEM^-$. The fetus was kept by a pincette, and the head was incised. Firstly, the mouth was cut sideward by a scissors, and the face was cut straight and thickly along the centerline from the mouth toward the brows and then along the Y-shaped line toward the ears. The scalp and the skull were removed by a sharp pincette, and the naked brain was dipped by a pincette and taken into a fresh $MEM^-$.

The taken-out brain was put to face the olfactory region leftwards and stripped of its meninges toward the right with a pincette. Then, the brain was turned over to remove superfluities from the backside. Finally all the blood vessels were removed.

The cerebral cortex was picked by a round pincette into a 35mm dish to which 2ml of dispase I (3U/ml) was added, and was placed in a $CO_2$ 5% and humidity 95% incubator at 37°C for 5 minutes. Then, Dnase (1ml) was added thereto, and the dish was put in the incubator for 10 minutes again. The resultant solution was sufficiently pipetted while being careful not to make bubbles by a Pasteur pipette having the top end rounded by burning, so that the cells might be free from any bundles. The upper inlet of 50ml tube was covered with a 70μm mesh, and the mesh was then wetted with an adequate amount of $MEM^-$. The cell suspension thus obtained was poured into the tube through the mesh, and about 2ml of $MEM^-$ was poured to wash the mesh. The mesh was removed, and the resultant was messed up to 30-40ml with $MEM^-$. The thus obtained sample was centrifuged at 1,000 rpm for 5minutes, and the supernatant was removed. The sample was messed up again to 30-40ml with $MEM^-$, pipetted and centrifuged again at 1,000 rpm. The supernatant was removed, and the sample was supplied with 30ml of $MEM^-$, and sufficiently pipetted, and then the cell number was countered by a cell counter. The cell suspension was inoculated in the PLL-coated dishes to be $1.0 \times 10^7$ cells/dish. The liquid was adjusted with $MEM^+$ to be a final volume of 10ml/dish, and the cells were cultured in an incubator. Medium change and passage were carried out in accordance with the schedule as shown in Table below.

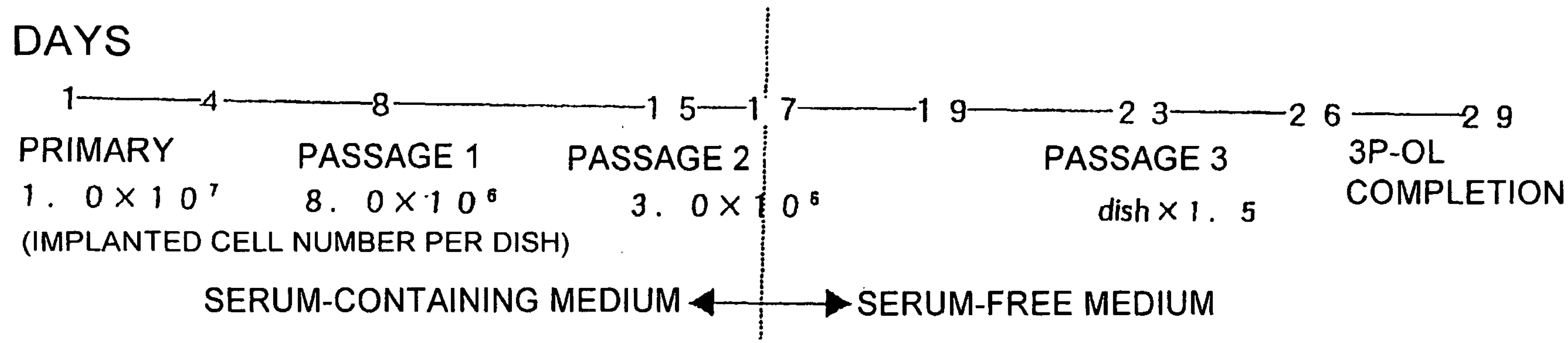


RED NUMERAL-DESCRIBED DAY: MEDIUM CHANGE WITH $MEM^+$

BLUE NUMERAL-DESCRIBED DAY: MEDIUM CHANGE WITH B.S

4TH DAY: MEDIUM CHANGE WITH $MEM^+$

FROM 17TH DAY: MEDIUM CHANGE WITH B.S (SERUM-FREE MEDIUM), AND PASSAGE WITH B.S

Medium change and passage were performed as follows.

(Medium change)

After disposal of the culture medium, the dish is washed twice with about 3ml of PBS(-), and 10ml of the fresh culture medium is poured. The medium change after the 17th day is carried out using B.S (serum-free medium). Only at the first medium change, 200µl of bFGF is added per dish. It is because the O-2A precursor cell is grown.

(Passage)

The dish is washed twice with about 5ml of PBS(-). At the first passage, the dish is left stand for 2-3 minutes after supplying PBS(-) at the second time. 2-3ml of 0.05% trypsin is added, and the dish is left stand in a 37°C incubator for 8 minutes. After observation of the cell condition with a microscope, while keeping the trypsin as it was, the dish is supplied with $MEM^+$ at the same amount as the trypsin, and the cells is pipetted to stop the action of trypsin. The resultant was centrifuged at 1,000 rpm for 5 minutes. After removing the supernatant, 20-30ml of $MEM^-$ is added, and the resultant is pipetted again, centrifuged at 1,000 rpm for 5 minutes. Then, the cell number is counted by a cell counter. The supernatant is discarded, 20-30ml of $MEM^+$ (B.S at the third passage) is added, and the resultant is pipetted, and the cell number is counted by a cell counter. Depending on respective passage stages, the cells are inoculated in respective dishes at the following number, and are cultured while left standing in an incubator.

1 st: $8.0 \times 10^6$

2nd: $3.0 \times 10^6$

3rd: dish × 1.5 (cell number)

(4) Experiment result

Fig. 6 shows the overview of the aforementioned culturing process and the result of the actual culturing.

In the initial 1 week, the sample is in a mixed state of various types of cells including, in addition to the O-2A precursor cell and a prooligodendrocyte (proOL), a neuron, an astrocyte and the like. However, by using trypsin in the passage, the highly trypsin-sensitive neuron is removed (see the result at the 1P-6th day in Fig. 6). The oligodendrocyte already matured at this stage also suffers from apoptosis to drop out.

The cell mixture was initially cultured in a MEM medium supplemented with fetus bovine serum (FBS). By replacing the medium with a serum-free medium (B.S) after the 2nd passage, the O-2A precursor cell is introduced in the oligodendrocyte lineage to make the astrocyte fall in death (see the result at the 2P-6th day in Fig. 6).

The above procedure could provide a culturing system at the 29th day where the oligodendrocytes were almost uniform in their differentiation stage and have a purity of 95% or more. (see the result of the 3P-OL in Fig. 6).

Example 3: Influence of cPA and LPA on cultured cells of oligodendrocytes

An experiment was carried out where cPA or LPA was added from the stage of primary culturing in the oligodendrocyte culturing system described in Example 2. Particularly, focus was put on how the survival, differentiation and proliferation of the cell would change when a lipid was added.

(1) Experiment method

The experiment procedure of cell culturing was same as in Example 2.

From the stage of primary culturing, cPA or LPA was added to be an amount of 5µM per dish every passage and medium change. The cPA derivative used in this experiment was an oleoyl cPA which was synthesized according to the method described in Example 1 by using oleoyl LPC as the substrate and using the Actinomyces *A.sp.*No. 362-derived PLD as the enzyme source.

In the case of medium change, cPA or LPA was added at the time of the replacement of a culture medium. In the case of passage, it was added after the cells were left stand in an incubator for 2-3 hours after the completion of passage to confirm that cells had adhered to the dish. Additionally, at every stage, the cells were subjected to

fluorescence straining and were monitored on the differentiation state of the cells through observation of a differentiation stage-specific expressed protein. Medium change and passage were carried out according to the following table.

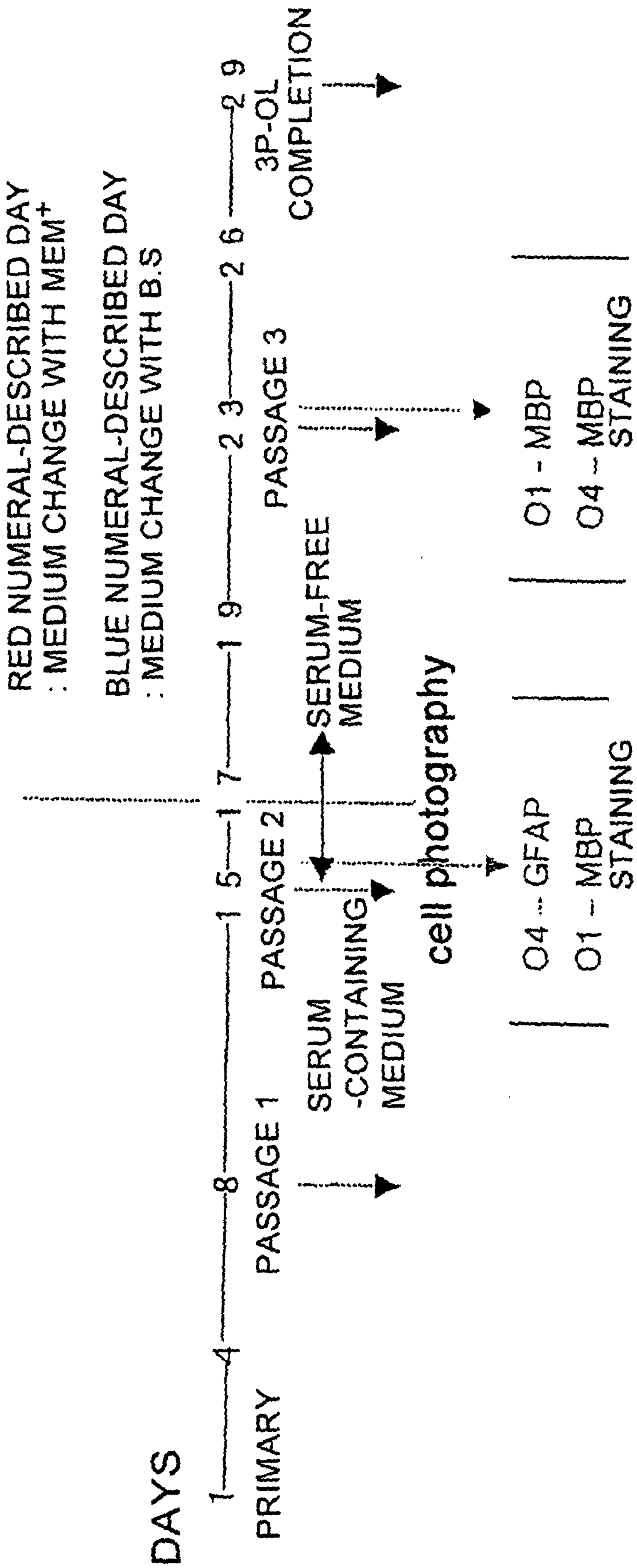
RED NUMERAL-DESCRIBED DAY
: MEDIUM CHANGE WITH MEM+
BLUE NUMERAL-DESCRIBED DAY
: MEDIUM CHANGE WITH B.S
DAYS
1
4
8
15
17
19
23
26
29
PRIMARY
PASSAGE 1
PASSAGE 2
PASSAGE 3
3P-OL
COMPLETION
SERUM
-CONTAINING
MEDIUM
SERUM-FREE
MEDIUM
cell photography
O4 -- GFAP
O1 -- MBP
STAINING
O1 - MBP
O4 -- MBP
STAINING

3P-OL was photographed and subjected to fluorescence straining every three days. The fluorescence straining of the 3P-OL was performed with a combination of the antibodies same as those described in Table 2. The method of the fluorescence staining will be described below.

The antibodies used for staining cells in this test example were four types of 01, 04, MBP, and GFAP. The expressed proteins are briefly explained.

04: expressed in oligodendrocytes from the early immature oligo stage to the final stage.

01: expressed in oligodendrocytes from the middle immature oligo stage to the final stage.

MBP (Myelin basic protein); expressed in oligodendrocytes from the latter immature oligo stage to the final stage (No MBP is expressed unless the oligodendrocyte grows near to a considerably mature stage. The expression is extended from the cell body to the dendrite, and therefore the farther the cell is stained toward the top of the dendrite, the more mature the cell is).

GFAP (Glial fibrillary acidic proteins): expressed in mature astrocytes.

The following Table 3 shows the protocols for fluorescent staining in 04-GFAP double staining and 01(04)-MBP double staining.

IN THE CASE OF O4-GFAP DOUBLE STAINING

FIXING WITH PLP FOR 15 MIN

PBS - wash THREE TIMES

blocking FOR 30 MIN

O4 (1 0 ×) FOR 45 MIN

PBS - wash SIX TIMES

Anti - Mouse - I gM(FITC) (2 0 0 ×) FOR 45 MIN

PBS - wash SIX TIMES

5% ACETIC ACID FOR 7 MIN
90% ETHANOL (-2 0 ℃)

PBS - wash THREE TIMES

GFAP (2 0 0 0 ×) FOR 45 MIN

PBS - wash SIX TIMES

Anti - Rabbit - I gG (Rhodamine) (2 0 0 ×) FOR 45 MIN

PBS - wash SIX TIMES

ENCLOSURE

IN THE CASE OF O1(O4)-MBP DOUBLE STAINING

FIXING WITH 4%-PFA FOR 15 MIN

PBS - wash THREE TIMES

blocking FOR 30 MIN

O1 (1 0 ×) (O4) FOR 45 MIN

PBS - wash SIX TIMES

Anti - Mouse - I gM(FITC) (2 0 0 ×) FOR 45 MIN

PBS - wash SIX TIMES

0. 5 %Triton FOR 30 MIN

PBS - wash THREE TIMES

MBP (1 0 ×) FOR 45 MIN

PBS - wash SIX TIMES

Anti - Rabbit - I gG (Rhodamine) (2 0 0 ×) FOR 45 MIN

PBS - wash SIX TIMES

ENCLOSURE

Upon staining, slide glasses were used. Slide glasses coated with PLL were put in a dish at the time of culturing. One of them was taken out upon staining, and the cells adhering thereto were stained.

(2) Experiment result

The cultured cells at the 6th day (P-6th day) from the primary culturing are shown in Fig. 7. It is understood that there is no large difference in cell appearance between the control and the LPA addition, but that the cells in the LPA addition aggregate to form bundles of the neurons. The bundled neurons appear to connect the aggregated cells with each other to keep their mutual contacts.

The cultured cells at the 6th day from the first passage (1P-6th day) are shown in Fig. 8. White grains found in Fig. 8 are the O-2A precursor cells, which are observed to increase in number more remarkably in the LPA addition and the cPA addition, particularly in the cPA addition, as compared with the control. In addition, it is observed that the cell density in the cPA addition is much higher than the others.

The cultured cells at the 6th day from the second passage (2P-6th day) are shown in Fig. 9. At this time, since the medium had been already replaced with the serum-free medium, the astrocytes in the control were weakened to be deformed into narrowly elongated ones and the cell density was not so high. The astrocytes in the LPA addition were also found to have been lowered in cell density, though not so wrong as in the control. On the contrary, the astrocytes in the cPA addition remained vigorous, and remained high in cell density as observed previously.

The cells which were stained and were photographed by a fluorescence microscope in order to observe the differentiation of the cells at this stage, are shown in Fig. 10. The upper shows a phase contrast image; the middle shows the cells stained with the 04 which is an antibody against the oligodendrocyte; and the lower shows the cells stained with the GFAP which is an antibody against the astrocyte. In the control, it is demonstrated that the astrocytes died ragged and the oligodendrocytes also could scarcely differentiate resulting in the short dendrites. In the LPA addition, the tissue of the astrocytes can be confirmed, and it is found from the photograph of 04 staining that the oligodendrocytes has the elongated dendrites to form fine mesh-shape and differentiations is proceeded. It can be confirmed that the astrocytes in the cPA addition exists in a dense tissue.

The cultured cells at the 6th day from the third passage (3P-6th day) are shown in Fig. 11. In the control, the state was that surplus cells have died and almost only the mature oligodendrocytes survive. On the contrary, in the cPA addition, the astrocytes which was deformed to the narrow ones still remain. In the LPA addition, it is found that little astrocytes remains, and that dendrites of the oligodendrocytes suffer from aoptosis and begin to be ragged.

The cells which were stained and photographed by a fluorescence microscope to observe the differentiation of the oligodendrocytes at this stage, are shown in Fig. 12. The upper shows a phase contrast image; the middle shows the cells stained with 04 which is an antibody against the oligodendrocyte; and the lower shows the cells stained with MBP which is an antibody against the oligodendrocyte. Both the 04 and MBP are the antibody against the oligodendrocyte. The 04 can stain even the oligodendrocyte at the early stage, while the MBP can satin only the oligodendrocyte at a highly differentiated stage.

MBP develops color stronger in the cPA addition and the LPA addition than in the control, and the color is developed fme up to the tip of dendrites. The expression of MBP begins with a cell body and extends to the tip of the dendrites. Therefore the farther the cell is stained toward the tip of the dendrite, the higher the cell differentiates. Particularly, it is found that, in the LPA addition, even the tip of the dendrite is stained.

Although an experiment ends with this stage in usual culturing, the cells were continuously cultured for further 10 days. The cultured cells are shown in Fig. 13. In ordinary circumstances, oligodendrocytes stretch its dendrite, which then coiled round an axon to form a myelin sheath. In other circumstances, apoptosis occurs from the tip of dendrite and the mesh-shaped dendrite falls ragged. This situation was clearly observed in the case of the LPA addition, where the cell number itself decreased and the dendrite fell ragged. In the cPA addition, on the contrary, it was observed that the astrocytes survived even at this stage, and that there were many dendrites of oligodendrocytes around them which were not suffered from apoptosis.

(3) Consideration

From the above experiments, it was revealed that cPA causes cells to aggregate and neurons to be bundled at the early culturing stage, that cPA promotes O-2A precursor cells to proliferate at the early culturing stage, and that cPA promotes the survival of astrocytes through all their culturing stages.

From the observation that cPA causes cells to aggregate and neurons to be bundled at the early culturing stage, it is considered that cPA promotes the contact between nerve cells, leading to their maintenance. It is considered that the cPA can promote the survival of the oligodendrocyte and can promote the formation of the myelin, by promoting the proliferation of O-2A precursor cells which will differentiate to oligodendrocytes..

Since astrocytes supply oligodendrocytes with nutrients, it is considered that astrocytes are greatly involved

in the promotion of the survival of oligodendrocytes. Therefore, it is considered that cPA promotes the survival of astrocytes and thereby maintains the supply of nutrients to oligodendrocytes, so that oligodendrocytes may survive longer.

It is known that, in a geriatric brain, glial cells are remarkably lost and myelins are damaged and decreased in number. As described above, it is considered that the cPA can retard senescence of brain through the promotion of the survival of oligodendrocytes which play a major role in myelin formation, and that it additionally can contribute to the recovery of the functions of damaged nerve cells.

INDUSTRIAL APPLICABILITY

According to the present invention, there is provided a medicament for treating a brain nerve disease, which can recover the functions of damaged nerve cells by promoting the proliferation and survival of oligodendrocytes that play a major role in myelin formation in cerebrovascular dementia such as senile dementia where glial cells are remarkably lost and particularly myelins are remarkably damaged and lost.

According to the present invention, there is provided a medicament that can promote the proliferation, differentiation and/or survival of glial cells which support the activity and maintenance of nerve cells, and neurological disorders associated with decrease of glial cells in number such as cerebrovascular dementia can be treated.

## Claims

1. A medicament for promoting the proliferation, differentiation and/or survival of glial cells, which comprises a cyclic phosphatidic acid derivative represented by the formula (I) as an active ingredient:

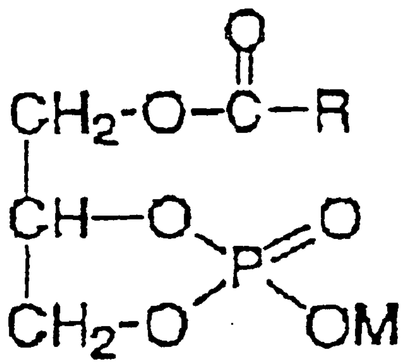

wherein R is a $C_{1-30}$ linear or branched alkyl group, a $C_{2-30}$ linear or branched alkenyl group, or a $C_{2-30}$ linear or branched alkynyl group, wherein these groups may contain a cycloalkane ring or an aromatic ring; and M is a hydrogen atom or a counter cation.

2. The medicament according to claim 1 wherein the glial cell is selected from an oligodendrocyte, a Schwann cell, an astrocyte or a microglia.

3. A medicament for treating and/or preventing a nerve disease associated with decrease of the glial cells which comprises a cyclic phosphatidic acid derivative represented by the formula (I) as an active ingredient:

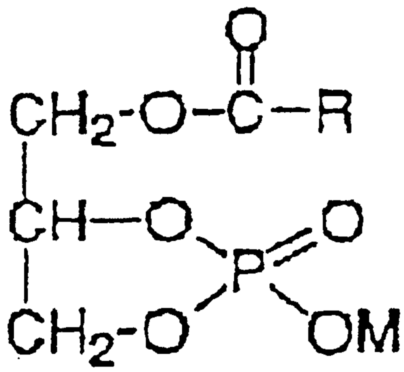

wherein R is a $C_{1-30}$ linear or branched alkyl group, a $C_{2-30}$ linear or branched alkenyl group, or a $C_{2-30}$ linear or branched alkynyl group, wherein these groups may contain a cycloalkane ring or an aromatic ring; and M is a hydrogen atom or a counter cation.

**4.** The medicament according to claim 3 wherein the nerve diseases associated with decrease of the glial cells is cerebrovascular dementia.

**5.** The medicament according to claim 4 wherein the cerebrovascular dementia is Binswanger's disease type dementia.

**6.** The medicament according to any of claims 1 to 5 wherein the cyclic phosphatidic acid derivative represented by the formula (I) is 1-oleoyl cyclic phosphatidic acid.

Fig1

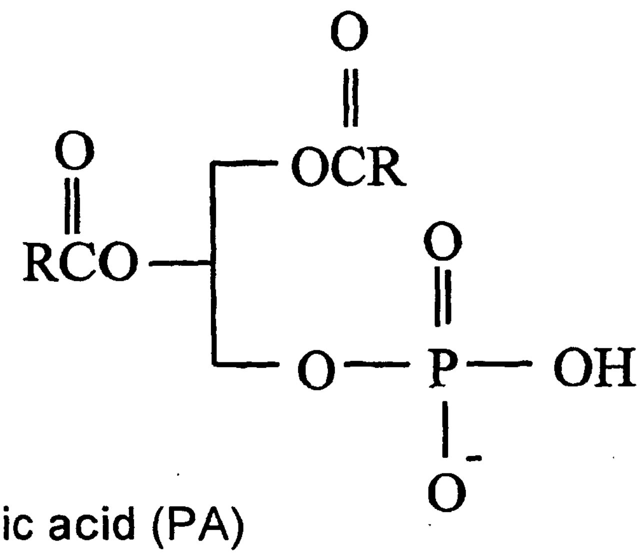

phosphatidic acid (PA)

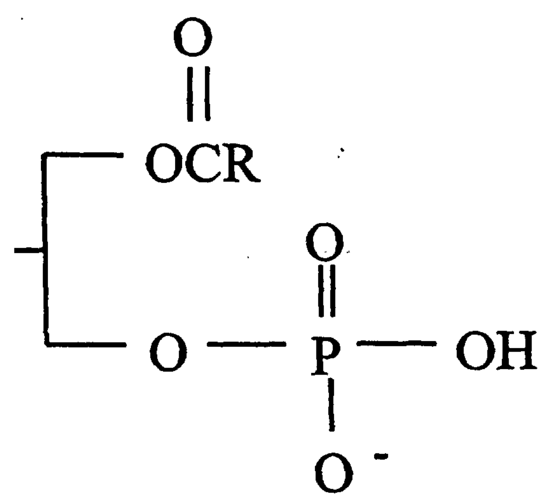

lysophosphatidic acid (LPA)

Fig2

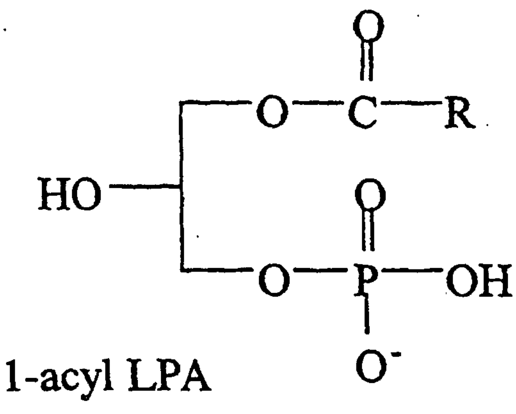

A. 1-acyl LPA

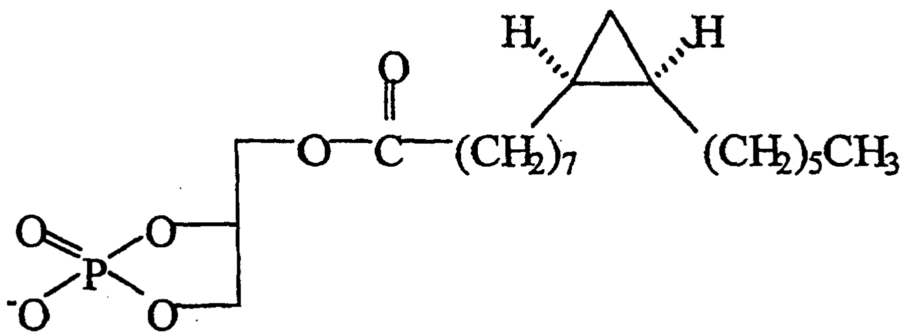

B. PHYLPA

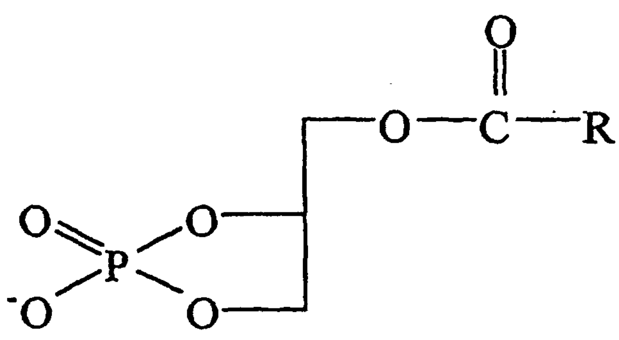

C. 1-acyl cPA

Fig3

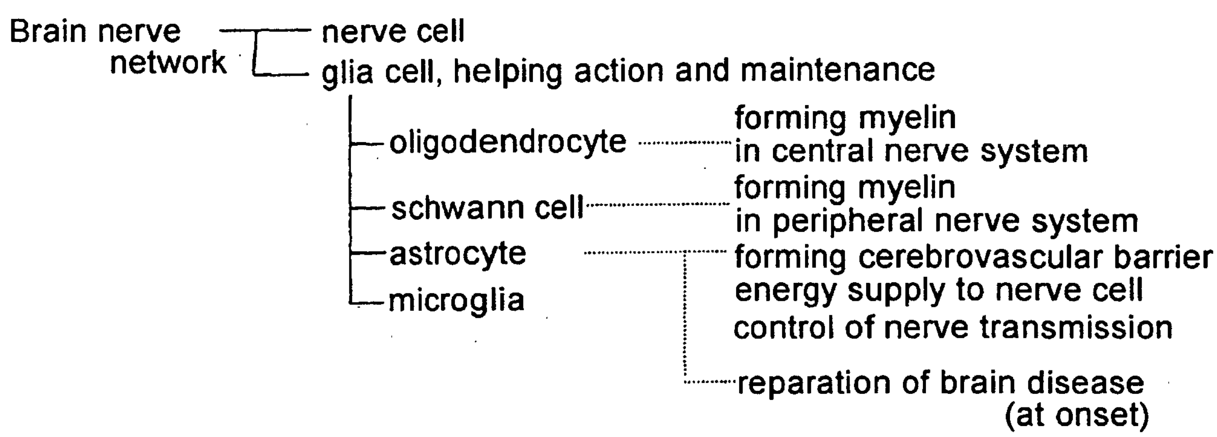
Brain nerve network
nerve cell
glia cell, helping action and maintenance
oligodendrocyte
forming myelin in central nerve system
schwann cell
forming myelin in peripheral nerve system
astrocyte
forming cerebrovascular barrier
energy supply to nerve cell
control of nerve transmission
microglia
reparation of brain disease (at onset)

Fig4

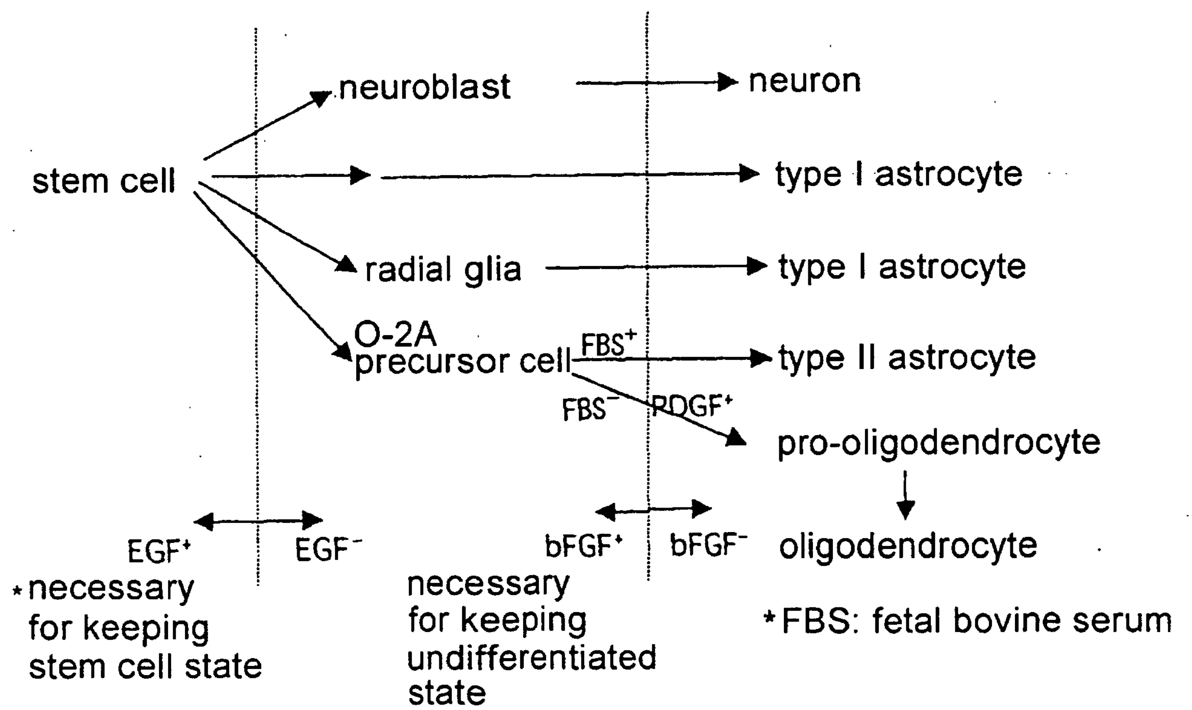
neuroblast
neuron
stem cell
type I astrocyte
radial glia
type I astrocyte
O-2A precursor cell
FBS+
type II astrocyte
FBS−
PDGF+
pro-oligodendrocyte
oligodendrocyte
EGF+
EGF−
*necessary for keeping stem cell state
bFGF+
bFGF−
necessary for keeping undifferentiated state
*FBS: fetal bovine serum

Fig5

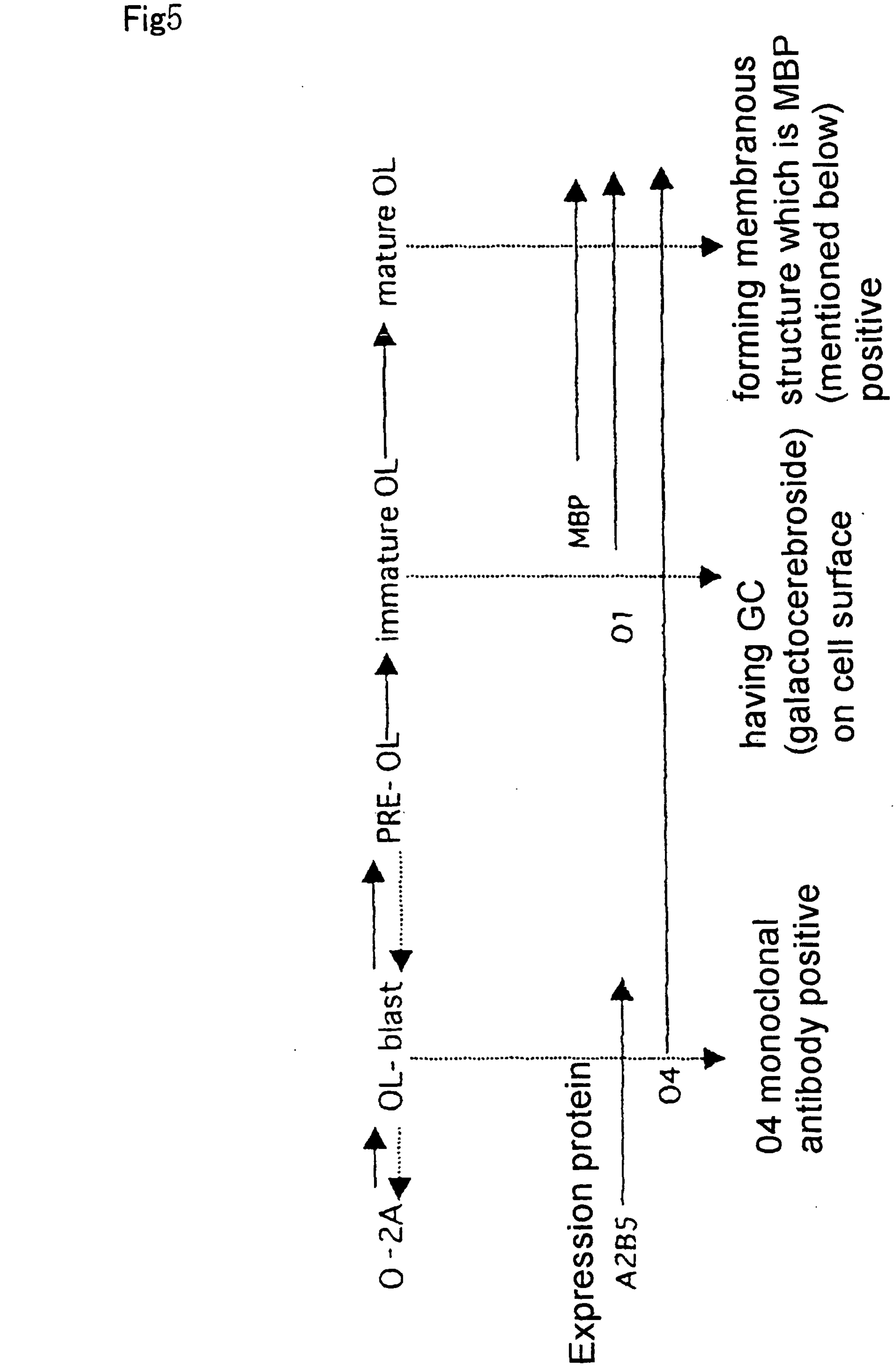
O-2A
OL-blast
PRE-OL
immature OL
mature OL
Expression protein
A2B5
04
01
MBP
04 monoclonal antibody positive
having GC (galactocerebroside) on cell surface
forming membranous structure which is MBP (mentioned below) positive

Fig6

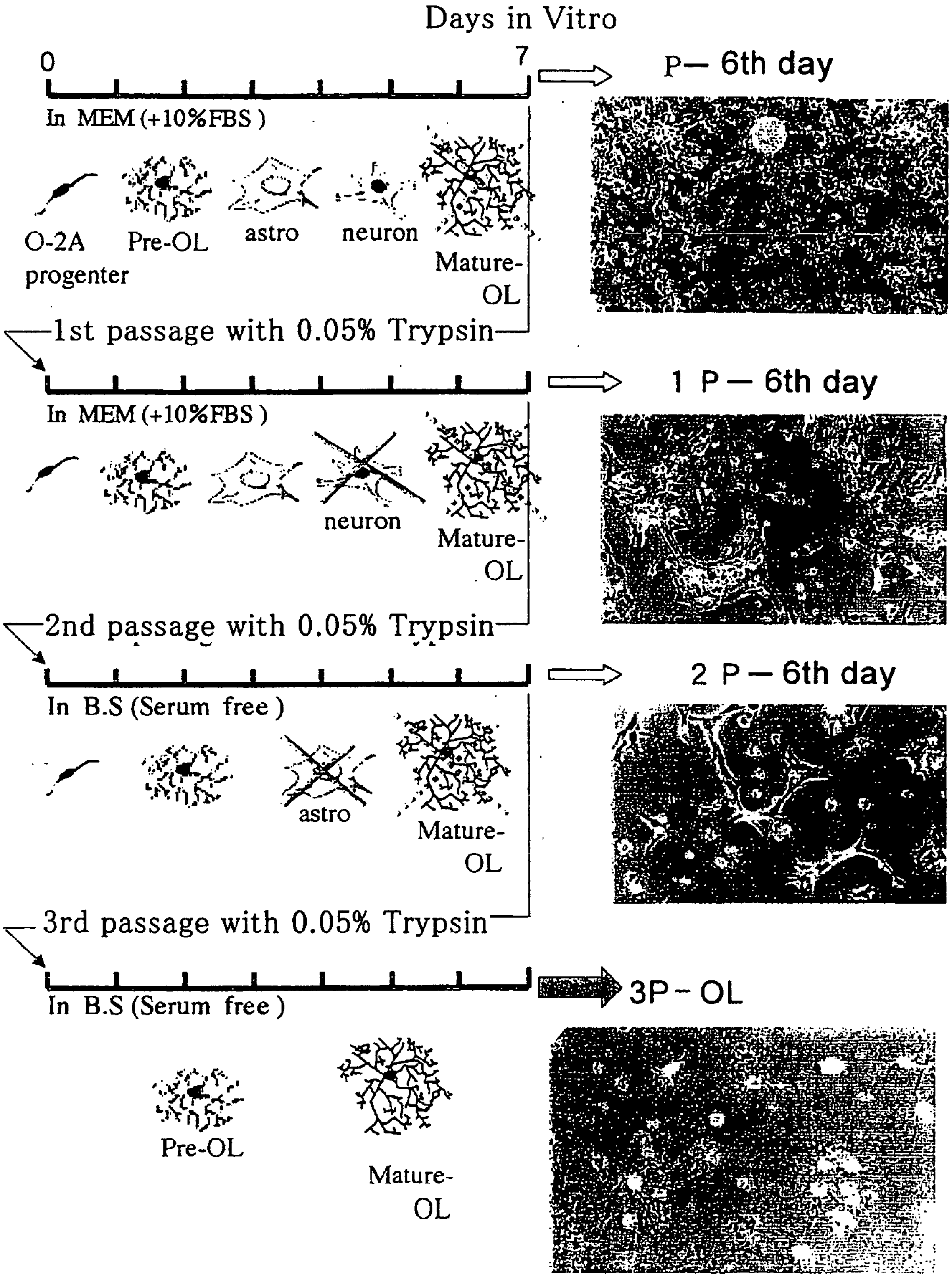
Days in Vitro
0
7
P– 6th day
In MEM (+10%FBS)
O-2A progenter
Pre-OL
astro
neuron
Mature-OL
1st passage with 0.05% Trypsin
1 P – 6th day
In MEM (+10%FBS)
neuron
Mature-OL
2nd passage with 0.05% Trypsin
2 P – 6th day
In B.S (Serum free)
astro
Mature-OL
3rd passage with 0.05% Trypsin
3P - OL
In B.S (Serum free)
Pre-OL
Mature-OL

Fig7

P - 6th day

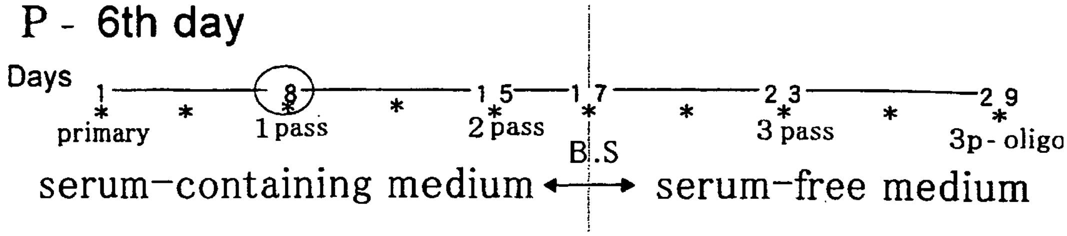


Control

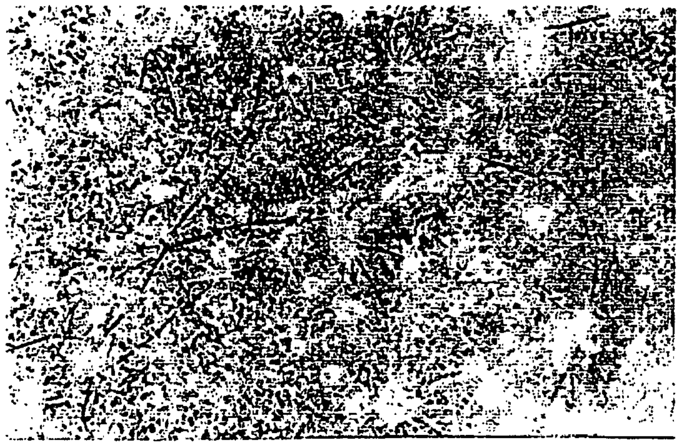

cPA addition

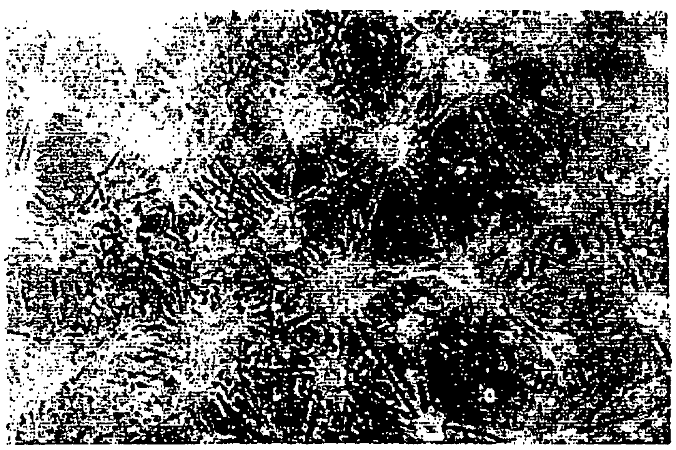

LPA addition

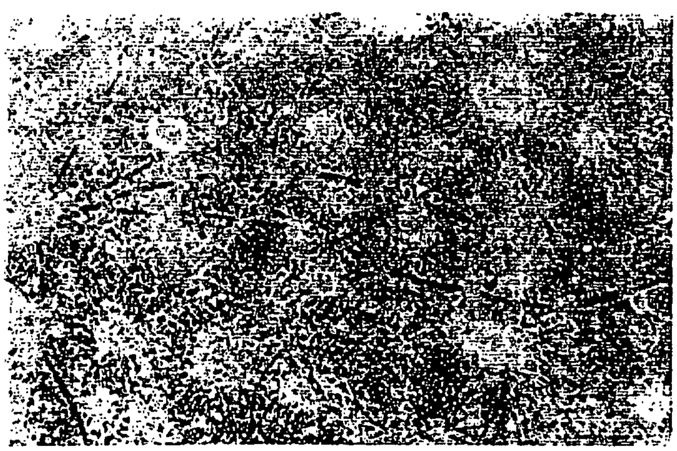

Fig8

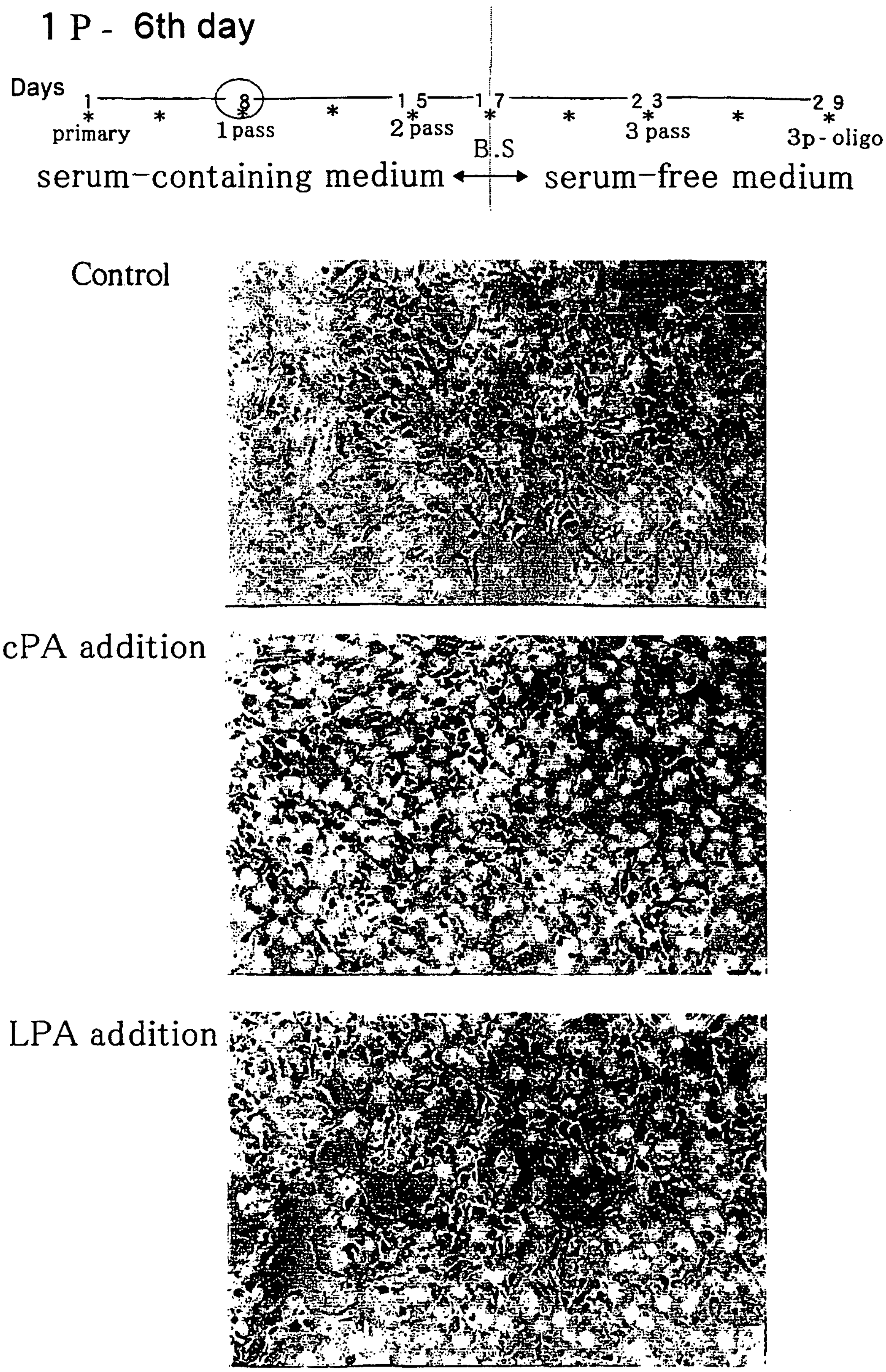
1 P - 6th day
Days
1
8
15
17
23
29
primary
1 pass
2 pass
B.S
3 pass
3p - oligo
serum-containing medium
serum-free medium
Control
cPA addition
LPA addition

Fig9

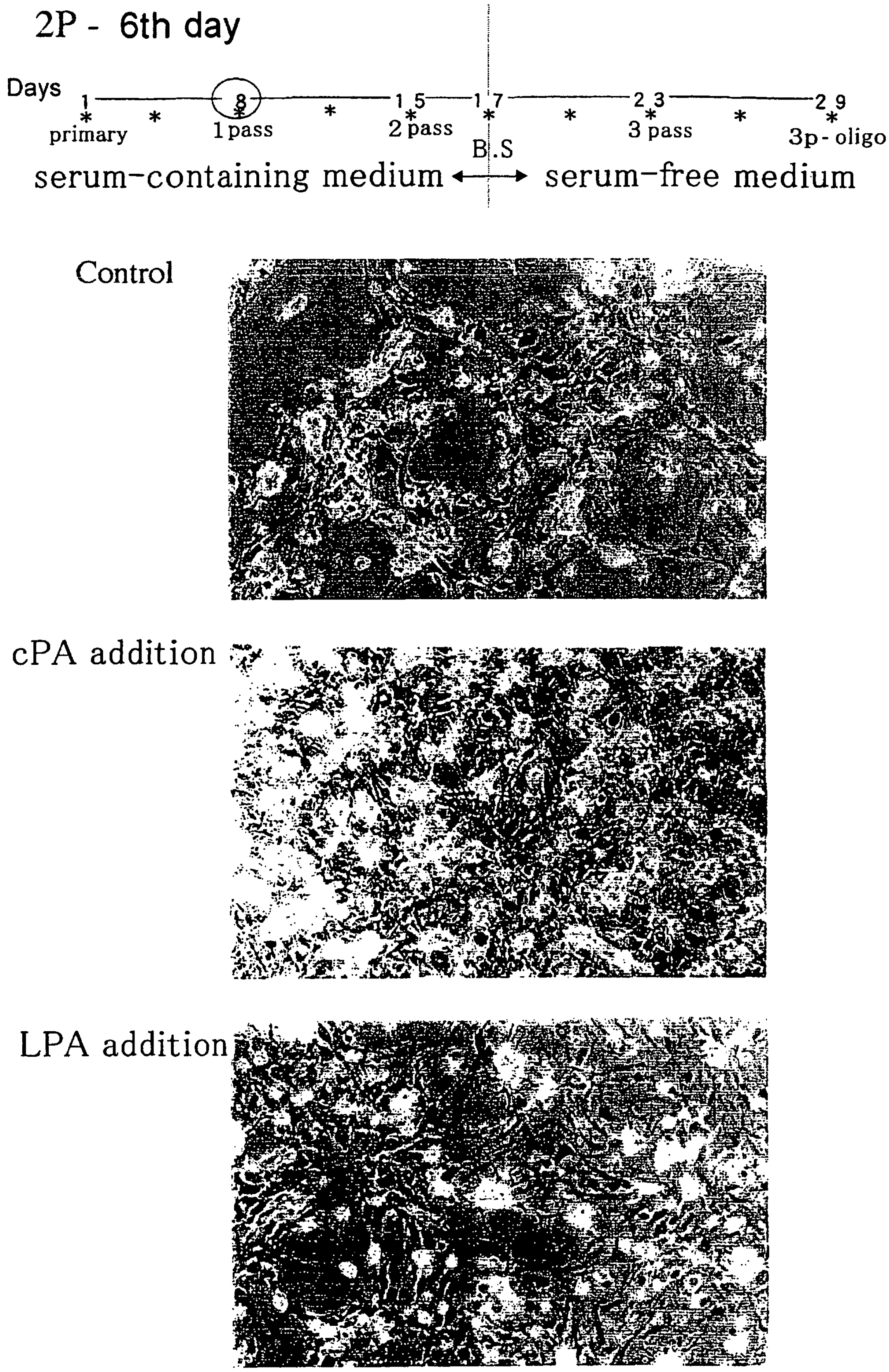
2P - 6th day
Days
1
primary
8
1 pass
15
2 pass
17
B.S
23
3 pass
29
3p-oligo
serum-containing medium
serum-free medium
Control
cPA addition
LPA addition

Fig10

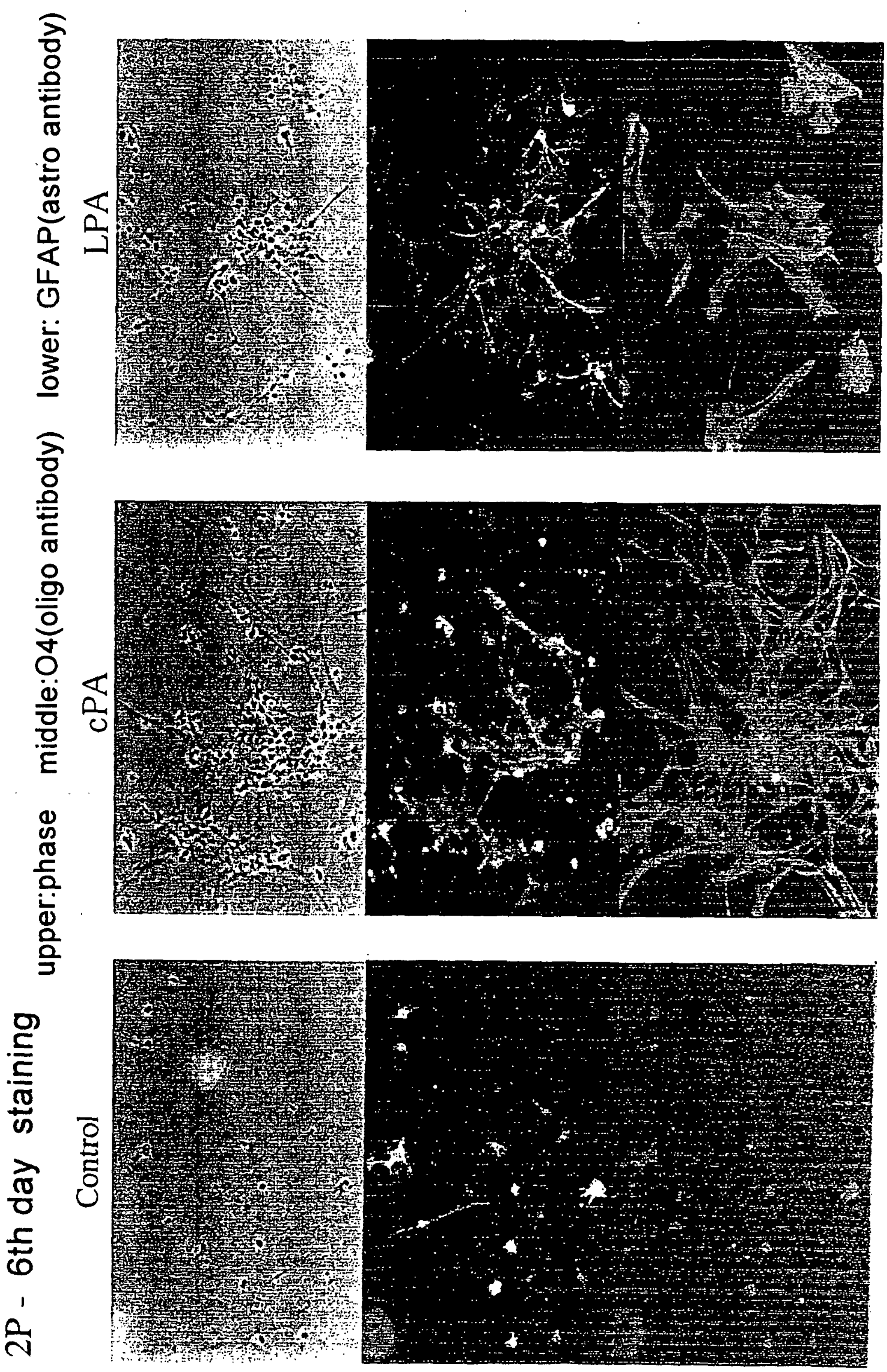
2P - 6th day staining
upper:phase middle:O4(oligo antibody) lower: GFAP(astro antibody)
Control
cPA
LPA

Fig11

3P - Oligo

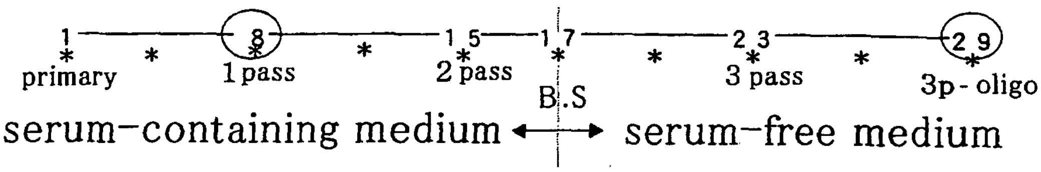


Control

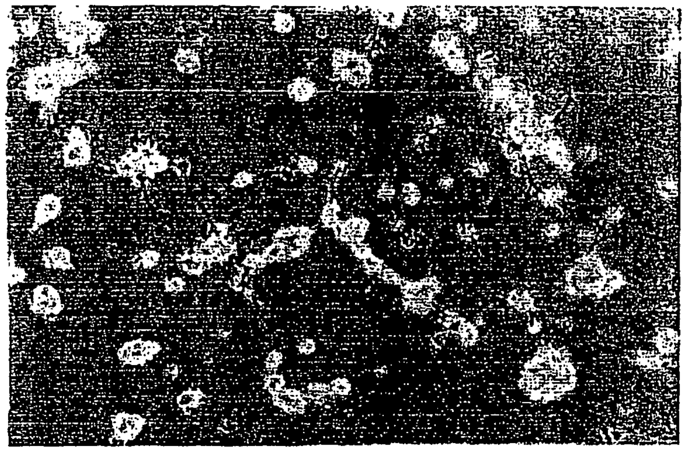

cPA addition

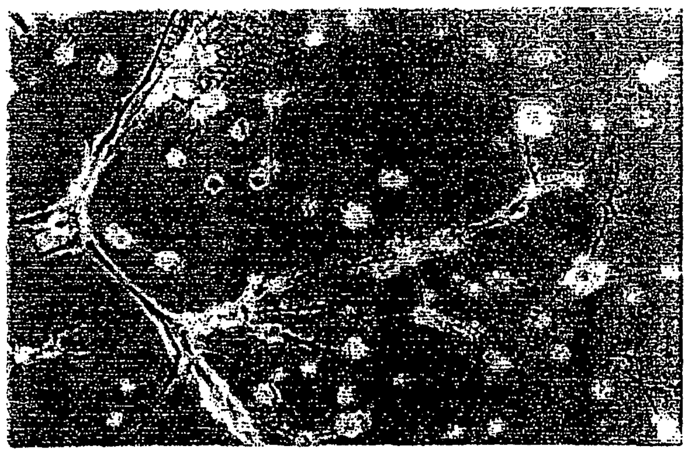

LPA addition

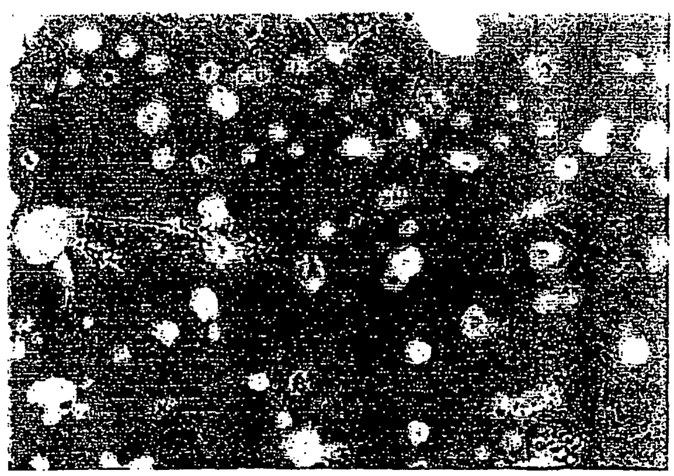

Fig12

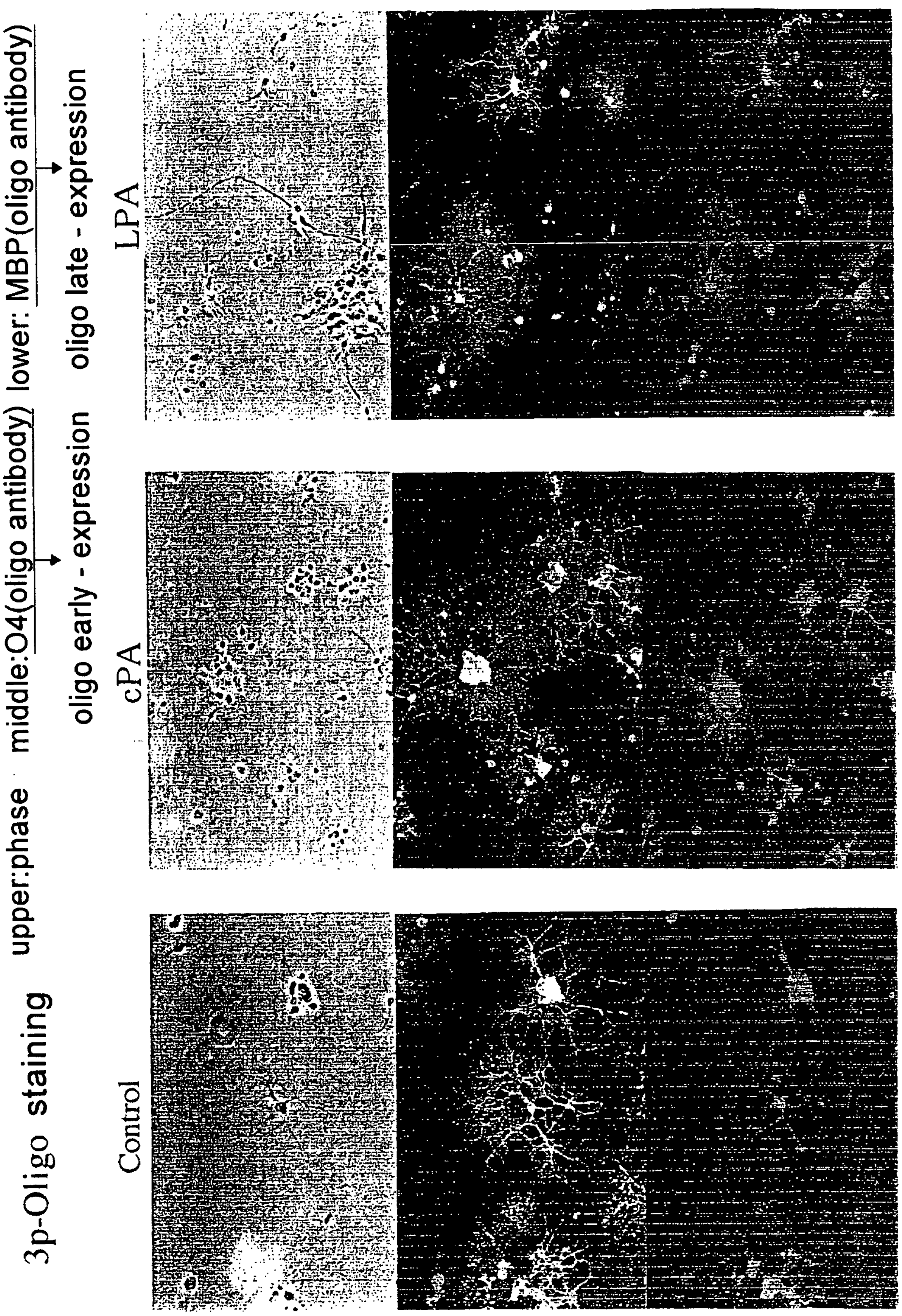
3p-Oligo staining
upper:phase
middle:O4(oligo antibody)
lower: MBP(oligo antibody)
oligo early - expression
oligo late - expression
Control
cPA
LPA

Fig13

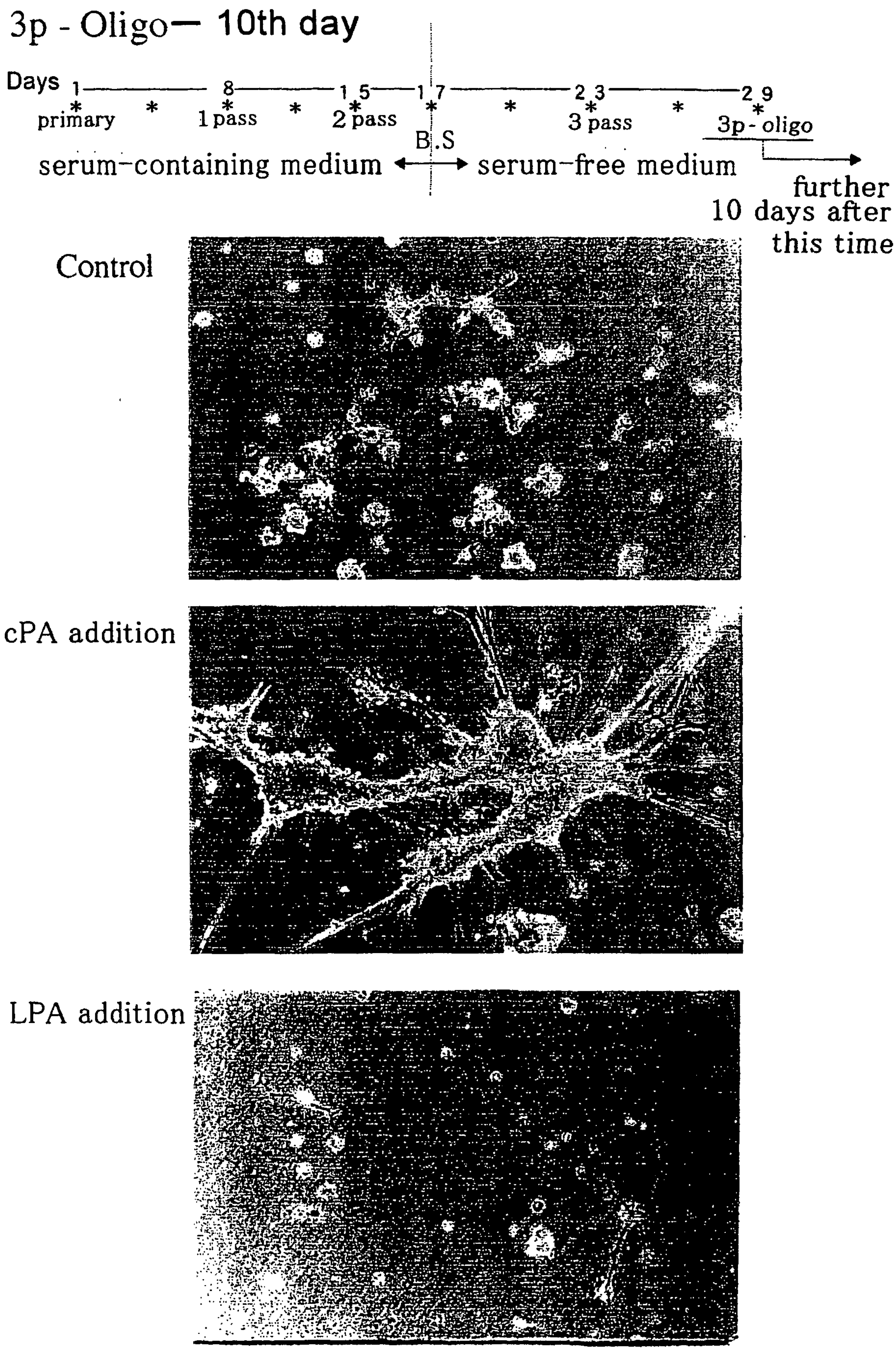
3p - Oligo— 10th day
Days 1 8 15 17 23 29
primary
1 pass
2 pass
B.S
3 pass
3p - oligo
serum-containing medium
serum-free medium
further 10 days after this time
Control
cPA addition
LPA addition

## INTERNATIONAL SEARCH REPORT

International application No.
PCT/JP02/03659

**A. CLASSIFICATION OF SUBJECT MATTER**

Int.Cl[7] A61K31/661, A61P25/00, 25/28, 43/00

According to International Patent Classification (IPC) or to both national classification and IPC

**B. FIELDS SEARCHED**

Minimum documentation searched (classification system followed by classification symbols)

Int.Cl[7] A61K31/66-668, A61P25/00-36, 43/00

Documentation searched other than minimum documentation to the extent that such documents are included in the fields searched

Electronic data base consulted during the international search (name of data base and, where practicable, search terms used)

CAPLUS(STN), CAOLD(STN), REGISTRY(STN), WPI/L(DIALOG), BIOSIS(DIALOG)

**C. DOCUMENTS CONSIDERED TO BE RELEVANT**

| Category* | Citation of document, with indication, where appropriate, of the relevant passages | Relevant to claim No. |
|---|---|---|
| Y | US 6150345 A (Regents of The University of Califolnia), 21 November, 2000 (21.11.00), Particularly, refer to the compounds stated in example 1 & WO 00/9139 A2 | 1-5 |
| Y | WO 00/57865 A2 (Yeda Research and Development Co., Ltd.), 05 October, 2000 (05.10.00), Particularly, refer to compound iii stated in Claims 1 and 13 & AU 3451700 A | 1-5 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 June, 2002 (10.06.02) | 25 June, 2002 (25.06.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/03659

C (Continuation). DOCUMENTS CONSIDERED TO BE RELEVANT

| Category* | Citation of document, with indication, where appropriate, of the relevant passages | Relevant to claim No. |
|---|---|---|
| Y | JP 7-14977 A (Sagami Chemical Research Center), 13 June, 1995 (13.06.95), Particularly, page 6 (Family: none) | 1-5 |
| Y | WO 99/47101 A2 (LXR Biotechnology, Inc.), 23 September, 1999 (23.09.99), Particularly, refer to the compound expressed by general formula (XI) shown on p.14 of the description; page 43, line 33 to page 45, line 15 & EP 1069895 A2 | 1-5 |

Form PCT/ISA/210 (continuation of second sheet) (July 1998)